# EUROPEAN PATENT SPECIFICATION

(11) **EP 4 260 864 B1**
(45) Date of publication and mention of the grant of the patent: **15.07.2026**
(21) Application number: 23167706.3
(22) Date of filing: 13.04.2023
(51) Int. Cl.: A61K 35/744, A61K 31/00, A61K 47/26, A61K 9/20, A61K 9/28, A61K 9/00, A61K 9/14, A61P 1/00, A61P 3/00, A61P 15/00, A61P 17/00, A61P 29/00, A61P 31/00

(54) **PRODUCT COMPRISING PROBIOTICS AND ISOMALTULOSE AND METHOD OF ITS PRODUCTION**
PROBIOTIKA UND ISOMALTULOSE ENTHALTENDES PRODUKT UND VERFAHREN ZU SEINER HERSTELLUNG
PRODUIT COMPRENANT DES PROBIOTIQUES ET DE L'ISOMALTULOSE ET SON PROCÉDÉ DE PRODUCTION

(30) Priority: 13.04.2022 EP 22168236
(43) Date of publication of application: 18.10.2023
(73) Proprietor: Bluestone Pharma GmbH, 6340 Baar (CH)
(72) Inventor: Gavrilovic, Danijela, 1600 Leskovac (RS); Dmitrovic, Milan, 11000 Belgrade (RS); Faber, Michael, 08870 Sitges (ES); Brandt, Karsten, 08870 Sitges (ES)
(74) Representative: Witthoff Jaekel Steinecke Patentanwälte PartG mbB

(56) References cited:
- CN-A- 105 104 765
- CN-A- 107 048 409
- CN-A- 110 051 003
- CN-A- 113 615 741
- US-B2- 8 282 976
- SU HUI-HUI ET AL: "Green synthesis of isomaltulose from cane molasses by an immobilized recombinant Escherichia coli strain and its prebiotic activity", LWT- FOOD SCIENCE AND TECHNOLOGY, vol. 143, 1 May 2021 (2021-05-01), United Kingdom, pages 111054, XP055950681, ISSN: 0023-6438, DOI: 10.1016/j.lwt.2021.111054
- YANG ZHAN-DONG ET AL: "Isomaltulose Exhibits Prebiotic Activity, and Modulates Gut Microbiota, the Production of Short Chain Fatty Acids, and Secondary Bile Acids in Rats", MOLECULES, vol. 26, no. 9, 23 April 2021 (2021-04-23), pages 2464, XP055950665, DOI: 10.3390/molecules26092464

## Description

### FIELD OF THE INVENTION

The invention relates to a compressed product, namely a lozenge, which comprises at least probiotic bacteria as active ingredient and isomaltulose as excipient, and wherein the compressed product is produced with a pressure of 6 to 10 kN/compressed product. Furthermore, the present invention is directed to a method for producing such a product with the indicated compression pressure as well as to the use of isomaltulose as excipient in the compressed product.

### BACKGROUND OF THE INVENTION

Probiotics are defined as live microorganisms which when administered in adequate amounts confer a health benefit on the host. The beneficial effects of probiotics may be mediated by a direct antagonistic effect against specific groups of undesired organisms, resulting in a decrease of their numbers, by an effect on the metabolism of such groups of organisms or by a general stimulatory effect on the immune system of animal or human hosts.

Probiotic microorganisms have been identified among microorganisms classified as yeasts, fungi and bacteria. For instance, lactic acid bacteria are in general recognized as being useful as probiotics or "probiotically active" organisms, *i.e.* organisms that may beneficially affect animal or human hosts.

Compressed tablets and powder formulations are the dominant dosage form for selfadministration of pharmaceutical compositions. Both products can be produced at a high speed and consequently at low prices and consumers are used to and frequently prefer these dosage forms. Furthermore, lozenges and powders are a particular preferred form especially for children since sucking a lozenge or administering a powder is regarded as more convenient than swallowing a tablet. Lozenges and powders are also particularly useful for the administration of probiotic bacteria conferring beneficial effects to the mouth microflora, like *Streptococcus salivarius,* since sucking of the lozenge (instead of mere swallowing) and administering a powder which dissolves in the mouth and thus, remains there for a certain time, allows the probiotic bacteria to colonize the oral cavity.

In this context, sugar free lozenges and powder products are becoming increasingly interesting for dietary reasons and for the sake of dental health. For example, in US 8,282,976 B2, a granulate instant mixture for a cacao beverage comprising probiotic bacteria and isomaltulose as sweetener and in CN 107 048 409 A, coated pellets comprising probiotics with isomaltulose and plant extracts for use in improving sleep are described. In CN 110 051 003 A, CN 105 104 765 A, and CN 113 615 741 A powder compositions comprising probiotics and isomaltulose as prebiotic are described.

Furthermore, the suggested minimal concentration for probiotic bacteria is 10⁶ CFU/g of a product (Shah (2000), J Dairy Sci 83(4):894-907) to provide health benefits, and thus, there is a constant need to provide healthy and consumer friendly products which contain a therapeutically effective amount of viable probiotically active organisms.

### SUMMARY OF THE INVENTION

The present invention relates to the embodiments as characterized in the claims. In particular, it has been surprisingly found that probiotically active organisms can be formulated with isomaltulose as excipient, in particular as bulking agent, wherein this composition can be formulated into tablets, in particular lozenges by compression in a tablet press with a pressure of 6 to 10 kN/compressed product , retaining sufficient amounts of probiotically active organisms.

The inventors have surprisingly shown that a lozenge comprising probiotic bacteria and isomaltulose instead of isomalt has superior properties that could not have been expected. In particular, as can be derived from the Examples, the CFUs of *S. salivarius* K12 are much higher in the isomaltulose lozenges than in the isomalt lozenges. Although the same concentration of freeze-dried *S. salivarius* K12 has been used as raw material, more viable bacteria are present in the isomaltulose lozenges in comparison to the corresponding lozenges with isomalt. Furthermore, the disintegration time of the isomaltulose lozenges is higher than the disintegration time of the isomalt lozenges and thus, the isomaltulose lozenges are sucked longer before they disintegrate than the isomalt lozenges, which has *i.a.* the advantage that there is more time for the oral probiotic to colonize the oral cavity. The friability of both lozenges is comparable and thus, both are stable. Furthermore, the average water activity of the isomaltulose lozenges is lower than the water activity of the isomalt lozenge and thus, isomaltulose lozenges have a longer shelf life.

In particular, as can be derived from Table 3, the isomaltulose lozenges comprise even after 12 months storage a CFU/g amount of probiotics which is higher than 10⁶ CFU/g which is regarded as the minimal concentration to provide health benefits (Shah (2000, *supra*)).*.*

Furthermore, the isomaltulose lozenges have a longer disintegration time than the isomalt lozenges, *i.e.* of more than 10 minutes in comparison to about 5 minutes, allowing an efficient colonialization of mouth probiotics in the oral cavity; see Tables 3, 4, 5 and 6. The lozenges also show a low water activity so that they can be regarded as stable and as having a long shelf life. The low friability which has been measured for the lozenges show that those have a high quality; see Tables 3 to 8.

Usually, frequently used excipients like fructose and maltodextrin are substituted with sugar alcohols, like isomalt to provide sugar free lozenges and powder products. However, also the consumption of isomalt has disadvantages, since for example an excessive consumption may have a laxative effect ("604. Isomalt (WHO Food Additives Series 20)"; Retrieved 2017-09-28; and Bachmann et al., (1984), Investigations of the metabolic effects of acute doses of Palatinit(R), Akt. Ernähr. 9 (1984), 65-70).

One advantage of isomaltulose is that - in comparison to sucrose and most other carbohydrates - it is "kind to teeth" since it is not a significant substrate for oral bacteria and thus, acid production from isomaltulose in the mouth is too slow to promote tooth decay. Another advantage of the use of isomaltulose as excipient for the production of compressed products is that the tablets can be pressed directly without the use of a binder and without controlled granulation as shown in the Examples.

Accordingly, the present invention provides a compressed product, namely a lozenge which is kind to teeth and consumer friendly. In particular, the present invention provides a compressed product, namely a lozenge, which comprises at least probiotic bacteria as active ingredient and isomaltulose as excipient and wherein the probiotic microorganisms are mixed with the isomaltulose, wherein the mixture is homogenized, and wherein the mixture is compressed to the compressed product, wherein compression is performed with a pressure of 6 to 10 kN/compressed product. The compressed product preferably comprises lactic acid bacteria which are in general recognized as being useful as probiotics or "probiotically active" organisms, *i.e.* organisms that may beneficially affect animal or human hosts.

The compressed product of the present invention comprises in a preferred embodiment bacteria of the genus *Streptococcus, Lactobacillus, Lactococcus, Bifidobacterium* and/or *Enterococcus,* or a mixture of any one thereof. Preferably, the compressed product of the present invention comprises bacteria selected from the group consisting of, but not limited thereto: *Streptococcus salivarius,* preferably strain K12 or strain ENT-K12, M18, 24SMB, Rosell^{®}-83, or HA-188, but more preferably strain K12, ENT-K12, or M18 and most preferably strain K12 and ENT-K12, respectively, *Lactobacillus rhamnosus,* preferably strain LGG, *Lactobacillus casei,* preferably strain 431, *Lactobacillus paracasei,* preferably strain LP-33 (also designated as GMNL-32 or GM-080) or strain GMNL-133, *Lactobacillus fermentum,* preferably strain LC40, *Lactobacillus crispatus,* preferably strain M247, *Bifidobacterium animalis subsp. lactis,* preferably strain BB-12, *Lactobacillus plantarum,* preferably strain 299v, *Lactococcus lactis, Enterococcus faecalis, Lactobacillus reuteri protectis,* and *Streptococcus dentisani,* or a mixture of any one thereof, preferably of two of the mentioned strains.

As it is commonly known in the art and shown in several clinical studies, probiotic organisms have a health benefitting effect and can even be used for the treatment of various diseases; see for example Wescombe et al., Future Microbiol. (2012) 7(12), 1355-1371; Zupancic et al., Probiotics Antimicrob Proteins (2017) 9(2), 102-110; Wilcox et al., Clin Microbiol Infect (2019) 25(6), 673-680; Marom et al., Medicine (Baltimore) (2016) 95(6), e2695; Clark, Curr Opin Immunol (2020) 66, 42-49; Bertuccioli et al., Nutrafoods (2019) 2, 80-88. Accordingly, the present invention further relates to the compressed product for use in the treatment of otitis, preferably otitis media; upper respiratory tract infections, preferably tonsillitis or pharyngitis; lower respiratory tract infections, preferably bronchitis or pneumonia; diseases and inflammations of the oral cavity, preferably caries, oral mucositis, periodontitis, candidiasis, and/or oral lichen planus; halitosis; skin disorders, preferably acne and/or dermatitis; gastrointestinal problems; allergies or immune diseases preferably allergic rhinitis, or mastitis in a subject.

Furthermore, the product is suitable for supporting healthy mouth microflora, healthy upper and lower respiratory tract microflora, healthy skin, improving weight management, maintaining healthy gut microflora, healthy vaginal flora; maintaining a normal digestion or supporting healthy gut mobility, bowel movement and/or healthy stool frequency, stool consistency and/or form in a subject.

The present invention further relates to a method of producing a product comprising probiotic bacteria and isomaltulose, wherein the method comprises at least mixing the probiotic bacteria with isomaltulose, optionally with further excipients and/or active ingredients. The method for producing a compressed product of the present invention further comprises the compression of the mixture with a pressure of 6 to 10 kN/compressed product. To guarantees a low water activity of the product, the excipients are dried before tablet pressing. Furthermore, during tablet pressing, the generation of excessive heat is avoided and pressing is performed with a pressure which allows the compaction of the mixture but without being lethal for the probiotic organism.

Further embodiments of the present invention will be apparent from the description, the Figures and Examples that follow.

### BRIEF DESCRIPTION OF THE DRAWINGS

- **Fig. 1:**: Exemplarily manufacturing flowsheet for compressed products comprising probiotics, in particular *S. salivarius* K12 and isomaltulose by dry compression.
- **Fig. 2:**: Exemplarily manufacturing flowsheet for compressed products comprising probiotics, in particular *S. salivarius* K12 and isomaltulose including a wet granulation step.

### DETAILED DESCRIPTION OF THE INVENTION

The present invention relates to a compressed product which comprises at least probiotic microorganism as active ingredient and isomaltulose as excipient, wherein the compressed product in a lozenge, also referred to as tablet herein, and wherein the compressed product is produced with a compression pressure of 6 to 10 kN/compressed product, in particular wherein the probiotic microorganisms are mixed with the isomaltulose, wherein the mixture is homogenized, and wherein the mixture is compressed to the compressed product, wherein compression is performed with a pressure of 6 to 10 kN/compressed product. In particular, the inventors found a generally useful probiotic composition in the form of a compressed product, namely a lozenge, comprising viable, probiotically active organisms, and a method for its production. As regards the compressed product, the inventors found a production method by which the probiotic tablet dosage form can be produced by dry compression in a tablet press comprising probiotically active organisms and isomaltulose as a bulking agent, wherein the compression was performed with 6 kN to 10 kN/compressed product. Furthermore, the experiments performed in accordance with the present invention showed that said probiotic tablet dosage form can also be produced by tablet pressing including a wet granulation step. Thus, the present invention also relates to the use of isomaltulose as excipient in the compressed product of the present invention.

When features/embodiments/characteristics are described in connection with "the product" or "the product of the present invention", those generally relate to the compressed product if not indicated otherwise. Thus, all features described in regard to "the product" or "the product of the present invention" are features which are attributed to the compressed product if not indicated otherwise.

The product of the present invention is a compressed product, namely a lozenge.

Tablets for use in the mouth like lozenges are a favored delivery form since especially children associate a lozenge with a candy and since sucking a tablet is generally more convenient than swallowing a whole tablet leading to a high acceptance of such a product. Thus, the compressed product of the present invention is a lozenge.

Tablets for use in the mouth are usually uncoated and they are usually formulated to effect a slow release and local action of the active ingredient(s) (for example, compressed lozenges) or the release and absorption of the active ingredient(s) under the tongue (sublingual tablets) or in other parts of the mouth (buccal) for systemic action.

Isomaltulose is a disaccharide carbohydrate composed of glucose and fructose and it tastes similar to sucrose (table sugar) with half the sweetness. The glucose and fructose are linked by an alpha-1,6-glycosidic bond (chemical name: 6-0-α-D-glucopyranosyl-D-fructose). Isomaltulose, also known by the trade name Palatinose, is manufactured by enzymatic rearrangement (isomerization) of sucrose from beet sugar. In European Patent 0 028 905 B1, the use of isomaltulose as diluent in tablets in generally described as well as methods of producing such tablets.

One advantage of isomaltulose is that - in comparison to sucrose and most other carbohydrates - it is not a significant substrate for oral bacteria. Consequently, acid production from isomaltulose in the mouth is too slow to promote tooth decay and thus isomaltulose is "kind to teeth". Fermentation of carbohydrates by bacteria in the mouth (especially on the teeth) is responsible for the formation of dental plaque and oral acids. The acid initiates tooth demineralization and tooth decay (dental caries). Isomaltulose largely resists fermentation by oral bacteria and is the first carbohydrate of its kind with negligible acid production on teeth, as shown by pH telemetry. The evidence is strong and provides the basis for "kind to teeth" claims approved by both the Food and Drug Administration in the USA and European authorities following a positive opinion from the European Food Safety Authority. In addition, the product of the present invention has been considered "toothfriendly" as determined by standardized *in vivo* pH-telemetry tests conducted by test facilities accredited by Aktion Zahnfreundlich (Switzerland). A product is considered "toothfriendly" if it lacks a significant cariogenic and erosive potential in healthy people under usual conditions of use.

This is of particular relevance for tablets for use in the mouth like the compressed product of the present invention.

Another advantage of the use of isomaltulose as a diluent for the production of compressed products is that the tablets can be pressed directly without the use of a binder and without controlled granulation. The method of production of the product of the present invention will be explained in detail further below.

A further favorable feature of the compressed product of the present invention, which seems to be attributable to the presence of isomaltulose, is its long disintegration time. In particular, the compressed product of the present invention has a longer disintegration time than corresponding tablets with isomalt as excipient as shown in the Examples. Due to this long disintegration time, the compressed product remains longer in the oral cavity during sucking. This is particular useful when probiotic bacteria are comprised in the compressed product which are for use in the treatment of upper and lower respiratory tract infections, diseases and inflammations of the oral cavity, and halitosis or which are suitable for supporting healthy mouth microflora, healthy upper and lower respiratory tract microflora since the longer sucking time allows the probiotic bacteria to efficiently colonize the oral cavity and the respiratory tract.

Since isomaltulose is "kind to teeth", the long sucking time of the compressed product has no negative effect on the health of teeth and even when the product comprises probiotic bacteria which rather act in the gastro-intestinal tract, the compressed product is used since for example lozenges rather reminds on a candy than on a medicament or health stimulating agent. Furthermore, for a fast majority of people, sucking a tablet is more convenient than swallowing a tablet.

Thus, in one embodiment, the compressed product of the present invention, which comprises at least 80 % isomaltulose and further comprises at least a lubricant/an anti-caking agent and an aromatizing agent; or a lubricant/an anti-caking agent, a binder and an aromatizing agent has a disintegration time of more than 5 minutes, preferably between 10 and 30 minutes, more preferably between 10 and 15 minutes and most preferably between 10 and 11 minutes. In comparison, a corresponding tablet comprising isomalt as excipient has a disintegration time of about 5 minutes; see the Examples.

Furthermore, the amount of viable probiotically active microorganisms, in particular of *Streptococcus salivarius,* was higher in tablets comprising isomaltulose as excipient than in corresponding tablets comprising isomalt although the same amount of bacteria has been used for producing the compressed products; see Example 1. Accordingly, the formulation of the present invention, *i.e.* the use of isomaltulose in the product of the present invention, has a beneficial effect on the survival of the probiotically active microorganisms.

As can be derived from Example 1 and Table 3, during manufacturing of the tablets comprising isomaltulose as excipient, the viability of the tablets decreased only from 1.30E+10 CFU/g and 1.04E+10 CFU/g, respectively prior to compression to 1.22E+10 CFU/g and 9.60E+09 CFU/g directly after compression, *i.e.*, in the freshly prepared tablet composition. This corresponds to a decrease in viability of the cells of only about 7 % in average during tablet pressing. Thus, the use isomaltulose as excipient in the compressed product of the present invention results in that the viability of the cells during compression in a tablet press does not decrease by more than 10 %, preferably by no more than 8 %, and in particular by no more than 7%. The decrease in viability of the cells is calculated as: ([the total number of viable cells present in 1 g of the mixture prior to compression] - [the total number of cells present in 1 g of the freshly prepared tablet composition]) divided by [the total number of viable cells present in 1 g of the mixture prior to compression], multiplied by [100%].

Furthermore, the compressed product of the present invention has a hardness (resistance to crushing) of about 70 to 85 N and 6 to 10 Kp, respectively, which is comparable to the hardness of corresponding isomalt tablets. Tablet hardness is the force (load) required to break a tablet. The compressed product of the present invention further has a friability of about 1.2 to 1.5 %, which is comparable to the friability of the corresponding isomalt tablets. Friability describes the tendency of a solid substance to break into smaller pieces under duress or contact.

The water activity of a product is important for its shelf-life. In particular, a low water activity ensures a high survival rate of the probiotic bacteria which is important for the effectiveness of the product of the present invention. The water activity of the compressed product of the present invention directly after compression is between about 0.1 and 0.2, preferably between 0.11 and 0.15 and in average of about 0.14 and slightly increases over time, and thus, the product has a high shelf-life. The water activity of the corresponding isomalt product is higher as can be derived from the Examples. Thus, the water activity of the product of the present invention is between 0.11 and 0.15 after production.

The International Scientific Association for Probiotics and Prebiotics defines "probiotics" as "live microorganisms that, when administered in adequate amounts, confer a health benefit on the host". These microorganisms, which consist mainly of bacteria but also include yeasts, are naturally present in fermented foods, may be added to other food products, and are available as dietary supplements, like the product of the present invention. In principle any probiotic strain can be formulated in the product of the present invention. Health benefits have mainly been demonstrated for specific probiotic strains of the following genera: *Aerococcus, Bacillus, Bacteroides, Bifidobacterium, Clostridium, Enterococcus, Fusobactehum, Lactobacillus, Lactococcus, Leuconostoc, Melissococcus, Micrococcus, Pediococcus, Peptostrepococcus, Propionibacterium, Staphylococcus, Streptococcus and Weissella.* Thus, the compressed product of the present invention, preferably comprises species of any one of those genera or mixtures thereof.

Common microorganisms which may be formulated in the product of the present invention include but are not limited to the following:
Lactic acid bacteria: Genus *Lactobacilli* spp.; Species: *Lactobacillus acidophilus, L. alimentarius, L. amylovorus, L. brevis, L. bulgaricus, L. casei, L. cellobiosus, L. crispatus, L. curvatus, L. delbrueckii* spp. *bulgaris, L. delbrueckii* spp. *lactis, L. farciminus, L. fermentum, L. gallinarum, L. helveticus, L. johnsonii, L. lactis, L. paracasei, L. plantarum, L. reuteri, L. rhamnosus, L. sake, L. salivarius*; Genus: *Streptococcus* spp. Species: *Streptococcus salivaris* spp., *S. salivarius (for example K12 or ENTK12), S. dentisani, S. faecalis, S. faecium;* Genus: *Lactococcus* ssp., Species: *L. lactis cremoris, L. lactis;* Genus: *Leuconostoc,* Species: *Lc. mesenteroides;* and Genus: *Pediococcus* spp., Species: *P. pentosaceus, P. acidilactici.*

Bifidobacteria: Genus: *Bifidobacterium* spp., Species: *B. adolescentis, B. animalis, B. bifidum, B. breve, B. essensis, B. infantis, B. laterosporum, B. thermophilum, B. longum.*

Propionibacteria: Genus: *Propionibacterium* spp., Species: *P. acidipropionici, P. freudenreichii, P. jensenii, P. thoenii.*

Enterobacteria: Genus: *Enterococcus* spp., Species: *E. fecalis, E. faecium. E. durans.*

Sporulated bacteria: Genus: *Bacillus* spp., Species: *B. alcolophilus, B. cereus, B. clausii, B. coagulans, B. subtilis.*

Other bacteria: Genus: *Escherichia coli,* Species: *E. coli*; Genus: *Sporolactobacillus* spp. Species: *S. inulinus.*

Yeasts: Genus: *Saccharomyces* spp., Species: *S. cerevisae (boulardii*); that isolated from litchi fruit in Indonesia have also been accepted and used as probiotics.

In a preferred embodiment, the product of the present invention comprises lactic acid bacteria or bifidobacteria, preferably those mentioned above. As used herein the term "lactic acid bacteria" refers to gram-positive, microaerophilic or anaerobic bacteria which ferment sugars with the production of acids including lactic acid as the predominantly produced acid, acetic acid, formic acid and propionic acid. Lactic acid bacteria are particularly preferred.

In a particular preferred embodiment, the probiotic bacteria are selected from the group consisting of: *Streptococcus salivarius,* preferably strain K12 (DSM 13084; BAA-1024), ENT-K12 (DSM 34540), 24SMB (DSM 23307), M18 (ATCC BAA-2593; DSM 14685), Rosell^{®}-83, or HA-188, but more preferably strain K12 or M18, and most preferably strain K12 and ENT-K12, respectively, *Lactobacillus rhamnosus,* preferably strain LGG (ATCC 53103; US 4,839,28*), Lactobacillus casei,* preferably strain 431 (ATCC 55544), *Lactobacillus paracasei,* preferably strain LP-33 (also designated as GMNL-32 or GM-080; CCTCC M 204012, US 6,994,848 B2) or strain GMNL-133 (CCTCC M 2011331, EP 2 581 461 B1), *Lactobacillus fermentum,* preferably strain LC40 (CECT5716, US 7,468,270 B2), *Lactobacillus crispatus,* preferably strain M247 (LMG P-23257, EP 1 930 018 A1), *Bifidobacterium animalis subsp. Lactis,* preferably strain BB-12 (DSM 15954, EP 2 990 045 B1), *Lactobacillus plantarum,* preferably strain 299v (DSM 9843), *Lactococcus lactis* (Zamfir et al. 2016 Int J Food Sci Technol, 51, 2164-2170), *Enterococcus faecalis* (DSM 16440), *Lactobacillus reuteri protectis,*(DSM 17938); *Streptococcus dentisani* (CECT7746), and/or any combinations thereof.

An accession number starting with "DSM" indicates that the strain is deposited with the Deutsche Sammlung von Mikroorganismen Und Zellkulturen GmbH (DSMZ), Mascheroder Weg lb, D-38124 Braunschweig, GERMANY; a number starting with "ATCC" indicates that the strain is deposited with the American Type Culture Collection (ATCC), P.O. Box 1549, Manassas, VA 20108, USA; a number starting with "CCTCC" indicates that the strain is deposited with the China Center for Type Culture Collection (CCTCC), College of Life Sciences, Wuhan University Wuhan 430072, China; a number starting with "CETC" indicates that the strain is deposited with the Colección Española de Cultivos Tipo (CECT), Edificio 3 CUE, Parc Cientific Universitat de Valencia, Catedrático Augustín Escardino 9, 46980 Paterna (Valencia); a number starting with "LMG" indicates that the strain is deposited with the BCCM/LMG Bacteria Collection, Laboratorium voor Microbiologie, Ghent University, K.L. Ledeganckstraat 35, B-9000 Ghent, Belgium.

In one preferred embodiment, the strain is *S. salivarius* K12, which is publicly available at the American Type Culture Collection (ATCC), P.O. Box 1549, Manassas, VA 20108, USA under Accession No. BAA-1024.

In one preferred embodiment, the strain is *S. salivarius* ENT-K12, which is genetically identical to *S. salivarius* K12 and which has been deposited at the Leibniz-Institut DSMZ - Deutsche Sammlung von Mikroorganismen und Zellkulturen GmbH, Inhoffenstraße 7B, 38124 Braunschweig, Germany, and assigned Accession Number DSM 34540 (Date of deposit: February 22, 2023). The depositor is the Probionet GmbH, Schützenstrasse 380, 9100 Herisau, Switzerland.

The invention is not, however, limited to these above-mentioned particular microorganisms. The person skilled in the art would recognize those microorganisms, which may be useful in the product according to the invention. A very important embodiment of the present invention is to combine two or more of the above mentioned probiotically active organisms, such as *e.g.* a preparation comprising a probiotically active *Lactobacillus* species and a probiotically active *Streptococcus* species.

The product of the present invention preferably comprises the following probiotically active organisms:
- *Streptococcus salivarius,* preferably K12, ENT-K12 or M18
- *Lactobacillus rhamnosus,* preferably strain LGG
- *Lactobacillus casei,* preferably strain 431
- *Lactobacillus paracasei,* preferably strain LP-33 and/or GMNL-133
- *Lactobacillus fermentum,* preferably strain LC40
- *Lactobacillus crispatus,* preferably strain M247
- *Lactobacillus plantarum,* preferably strain 299v
- *Bifidobacterium animalis subsp. lactis,* preferably strain BB-12
- *Lactococcus lactis*
- *Streptococcus salivarius* and *Lactobacillus rhamnosus,* preferably *Streptococcus salivarius* K12 or ENT-K12, and *Lactobacillus rhamnosus* strain LGG;
- *Streptococcus salivarius* and *Lactobacillus casei,* preferably *Streptococcus salivarius* K12 or ENT-K12, and *Lactobacillus casei 431*;
- *Streptococcus salivarius* and *Lactobacillus fermentum,* preferably *Streptococcus salivarius* K12 or ENT-K12, and *Lactobacillus fermentum* strain LC40;
- *Streptococcus salivarius* and *Lactobacillus crispatus,* preferably *Streptococcus salivarius* K12 or ENT-K12, and *Lactobacillus crispatus* strain M247;
- *Streptococcus salivarius* and *Lactobacillus plantarum,* preferably *Streptococcus salivarius* K12 or ENT-K12, and *Lactobacillus plantarum* strain 299v;
- *Streptococcus salivarius* and *Bifidobacterium animalis subsp. lactis,* preferably *Streptococcus salivarius* K12 or ENT-K12, and *Bifidobacterium animalis subsp. lactis* strain BB-12;
- *Streptococcus salivarius* and *Lactococcus lactis,* preferably *Streptococcus salivarius* K12 or ENT-K12, and *Lactococcus lactis;*
- *Streptococcus salivarius* and *Lactobacillus paracasei,* preferably *Streptococcus salivarius* K12 or ENT-K12, and *Lactobacillus paracasei* LP-33;
- *Streptococcus salivarius* and *Lactobacillus paracasei,* preferably *Streptococcus salivarius* K12 or ENT-K12, and *Lactobacillus paracasei* GMNL-133.

In one embodiment, any of the above-mentioned combinations which include a *Streptococcus salivarius* strain include *Streptococcus salivarius* K12.

In one embodiment, any of the above-mentioned combinations which include a *Streptococcus salivarius* strain include *Streptococcus salivarius* ENT-K12.

Of course, combinations any other of the above-mentioned strains and/or of three, four or more of the above-mentioned strains are also possible and encompassed.

Next to the probiotic microorganisms and isomaltulose, the product of the present invention comprises in one embodiment one or more further excipients and/or active ingredients.

The further active ingredients can be compounds that support and/or boost the immune system like vitamins and minerals. Those include, but are not limited to vitamin A, vitamin D3, vitamin B6, vitamin C, folic acid, vitamin B12, iron, zinc, copper, selenium and combinations thereof. In a preferred embodiment, the further active ingredient is vitamin D3 (cholecalciferol), for example in a form of a premix.

Excipients are helpful and/or necessary to prepare solid dosage forms. They are widely involved in the flow properties of mixtures, compression properties in tablet preparation, disintegration properties for smooth disintegration, sticking properties in which powder sticks to the surface of tablet compression punches to cause defects on the tablet surface, capping in which the upper portion of tablets is peeled off in a cap shape during compression, lamination in which a tablet is peeled off in a layered fashion, and binding properties for enhancing the hardness of tablets.

Excipients include bulking agents, diluents, carrier, binders, lubricants/anti-caking agents, disintegrators, preservatives, colors or flavors. The excipients are preferably pharmaceutically acceptable and/or acceptable for human consumption.

Acceptable carrier/bulking agents suitable for use in the administration of viable probiotic microorganisms are well known to those skilled in the art; see, for example, Remington's Pharmaceutical Sciences, 18th ed., Gennaro, ed., 1990, Mack Publishing Co., Easton, Pa., Preferably, the carrier/bulking agent is a pharmaceutically acceptable carrier/bulking agent and/or a carrier/bulking agent acceptable for human consumption. Acceptable carriers/bulking agents suitable for use with probiotically active microorganisms in the compressed product of the present invention are usually solid carriers known in the art and include, but are not limited to magnesium carbonate; magnesium stearate; celluloses; talc; sugars such as fructose, sucrose, mannitol, sorbitol, xylitol, lactose; sugar substitutes such as isomalt; starches; maltodextrin; flours; (fructose-)oligosaccharides and skim milk, and similar edible powders, but are not limited thereto.

Typical diluents, by way of example, are: starches; lactose; mannitol; kaolin; calcium phosphate or sulphate; inorganic salts such as sodium chloride; and powdered sugars and sugar substitutes as mentioned above or celluloses.

Typical binders include starch; gelatin; sugars such as lactose, fructose, and glucose; and the like. Natural and synthetic gums are also convenient, including acacia; alginates; locust bean gum; methylcellulose, e.g., Hydroxypropyl methylcellulose (HMPC); polyvinylpyrrolidine (PVP) tragacanth; PVP K-30; PVP K-25; xanthan gum: and the like. Polyethylene glycol (PEG 4000 or PEG 6000); ethyl cellulose; and waxes can also serve as binders as well as Nu-BIND^{®} and CompactCel^{®}DIS.

Lubricants and anti-caking agents to prevent sticking during formulation and to prevent the formation of lumps include slippery solids such as talc, silica, magnesium and calcium stearate, polyethylene glycol, stearic acid, hydrogenated vegetable oils, rice extract blend, for example Nu-MAG^{®}, CompactCel^{®}LUB, in particular CompactCel^{®} F clear 290.02 LUB, oat fiber blend, for example CompactCel^{®} F 200.28 LUB, potato starch, gum Arabica, CompactCel^{®}FLO, Nu-FLOW^{®}, silicon dioxide, tricalcium phosphate, and rice hulls, for example Nu-FLOW^{®}, or CompactCel^{®}FLO.

Disintegrators are substances which swell when wetted to break up the composition and release the *S. salivarius* or extract. The disintegrators include starches; clays; celluloses; algins and gums; more particularly corn and potato starches; methylcellulose; agar; bentonite; wood cellulose; cation exchange resins; alginic acid; guar gum; citrus pulp; carboxymethylcellulose; powdered sponge; silica; and sodium lauryl sulfate.

Aromatizing agents are known to the person skilled in the art and can be of any kind which give a good (or at least a different taste) to the compressed product. Those flavors include, but are not limited to strawberry, mint, orange, banana, passionfruit, cocoa, menthol, yuzu, lemon and/or combinations thereof, preferably passionfruit, cocoa and menthol; yuzu and mint; orange and mint.

In one embodiment, the compresses product of the present invention further comprises a lubricant/anti-caking agent and optionally a flavor. This relates in particular to a compressed product produced by dry compression. In one embodiment, the compressed product of the present invention further comprises a lubricant/anti-caking agent and a binder and optionally a flavor. This relates in particular to a compressed product produced by compression including a wet granulation step

In order to obtain a sweet taste of the product of the present invention, the compressed products further comprise stevia. Stevia is a natural sweetener and sugar substitute derived from the leaves of the plant species *Stevia rebaudiana.* Thus, in one embodiment, the product of the present invention further comprises a natural sweetener, preferably stevia.

In recent years, as consumer interest in well-being and natural foods has increased, the use of natural additives in place of synthetic additives in the food industry has gradually increased as well as the use of sugar substitutes. In particular, in pharmaceutical drugs and health functional foods, safety and environmentally friendly factors in production processes are considered important, and thus many products containing natural components are being developed and the market size thereof is also increasing.

Accordingly, in a preferred embodiment, the product of the present invention does not comprise a sugar, but only the sugar substitute isomaltulose, and optionally the natural sweetener stevia. Furthermore, in one embodiment the compressed product of the present invention does comprise as few additives as possible and thus, only further comprises (next to isomaltulose and the probiotic bacteria) a lubricant/anti-caking agent as excipient, but no other excipients like binders or disintegrators or does even comprise no further excipients. In particular, in one embodiment the compressed product of the present invention only further comprises a lubricant/anti-caking agent as excipient, but no other excipients like binders or disintegrators.

In one embodiment, the compressed product of the present invention does comprise as few additives as possible and thus, only further comprises (next to isomaltulose and the probiotic bacteria) a lubricant/anti-caking agent and a binder as excipients, but no other excipients like disintegrators.

However, the product of the present invention may comprise an aromatizing agent, preferably a natural flavor and optionally a natural sweetener, like stevia.

In a preferred embodiment, the lubricant/anti-caking agent which is comprised in the product of the present invention is magnesium stearate, or a natural replacement. Natural lubricants are known in the art and are for example a crude fat-containing bean powder as described in WO 2013/165131 A1, a rice extract blend, for example the product Nu-MAG^{®}, or the product CompactCel^{®} LUB (CompactCel^{®} F clear 290.02 LUB, Biogrund GmbH, Huenstetten, Germany), an oat fiber blend, for example the product CompactCel^{®} LUB (CompactCel^{®} F 200.28 LUB, Biogrund GmbH, Huenstetten, Germany) or further products, like potato starch, or gum Arabica. In another preferred embodiment, the lubricant/anti-caking agent which is comprised in the product of the present invention is silicon dioxide or tricalcium phosphate or a natural replacement. Natural anti-caking agents are also known in the art and are for example powdered cellulose, like JELUCEL^{®}, native potato starch, inulin, rice fibers, or rice hulls, for example Nu-FLOW^{®}, or CompactCel^{®}FLO. In another preferred embodiment, the binder comprised in the product of the present invention is HMPC or a natural replacement. Natural binders are for example CompactCel^{®}DIS, NuBind^{®}, or pregelatinized corn starch.

In a preferred embodiment, the compressed product of the present invention comprises any one of the probiotic bacteria or combinations of probiotic bacteria listed above, preferably *S*. *salivarius* K12 and ENT-K12, respectively, or *S. salivarius* M18 and a Lactobacillus strain, in particular *S. salivarius* K12 and *L. rhamnosus* LGG, or *S. salivarius* K12 and *L. casei* 431, or *S. salivarius* ENT-K12 and *L. rhamnosus* LGG, or *S. salivarius* ENT-K12 and *L. casei* 431, as well as isomaltulose, magnesium stearate and a flavor preferably selected from those mentioned above, most preferably strawberry flavor. Alternatively, magnesium stearate is substituted with a natural lubricant/anti-caking agent, preferably with an oat fiber blend, preferably comprising oat fibers, microcrystalline cellulose and sunflower oil refined, for example the product CompactCel^{®} F 200.28 LUB, or with a rice extract blend, preferably comprising rice extract, microcrystalline cellulose, and sunflower oil refined, for example the product CompactCel^{®} F clear 290.02 LUB, or any one of Nu-MAG^{®}, potato starch, gum Arabica, or with rice hulls, like Nu-FLOW^{®}, or CompactCel^{®} FLO, but most preferably with a rice extract blend in the compressed product of the present invention.

Accordingly, in one embodiment, the compressed product of the present invention comprises any one of the probiotic bacteria or combinations of probiotic bacteria listed above, preferably *S. salivarius* K12 and *S. salivarius* ENT-K12, respectively, or *S. salivarius* K12 and *S*. *salivarius* ENT-K12, respectively, and a Lactobacillus strain, in particular *S. salivarius* K12 and *S. salivarius* ENT-K12, respectively, and *L. rhamnosus* LGG, or *S. salivarius* K12 and *S*. *salivarius* ENT-K12, respectively, and *L. casei* 431, as well as isomaltulose, a rice extract blend, preferably comprising rice extract, rice hulls, gum arabic and sunflower oil, for example the product Nu-MAG^{®}, and the above-mentioned flavor, preferably strawberry flavor, and optionally, but preferably a natural sweetener, like stevia.

In one embodiment, the compressed product of the present invention comprises any one of the probiotic bacteria or combinations of probiotic bacteria listed above, preferably *S. salivarius* K12 and *S. salivarius* ENT-K12, respectively, or *S. salivarius* K12 and *S. salivarius* ENT-K12, respectively, and a Lactobacillus strain, in particular *S. salivarius* K12 and *S. salivarius* ENT-K12, respectively, and *L. rhamnosus* LGG, or *S. salivarius* K12 and *S. salivarius* ENT-K12, respectively, and *L. casei* 431, as well as isomaltulose, a rice extract blend, preferably comprising rice extract, microcrystalline cellulose and sunflower oil refined, for example the product CompactCel^{®}LUB, and the above-mentioned flavor, preferably strawberry flavor, and optionally, but preferably a natural sweetener, like stevia.

In one embodiment, the compressed product of the present invention comprises any one of the probiotic bacteria or combinations of probiotic bacteria listed above, preferably *S. salivarius* K12 and *S. salivarius* ENT-K12, respectively, or *S. salivarius* K12 and *S. salivarius* ENT-K12, respectively, and a Lactobacillus strain, in particular *S. salivarius* K12 and *S. salivarius* ENT-K12, respectively, and *L. rhamnosus* LGG, or *S. salivarius* K12 and *S. salivarius* ENT-K12, respectively, and *L. casei* 431, as well as isomaltulose, an oat fiber blend, preferably comprising oat fiber, microcrystalline cellulose and sunflower oil refined, for example the product CompactCel^{®}LUB, and the above-mentioned flavor, preferably strawberry flavor, and optionally, but preferably a natural sweetener, like stevia.

In another preferred embodiment, the compressed product of the present invention comprises any one of the probiotic bacteria or combinations of probiotic bacteria listed above, preferably *S. salivarius* K12 and *S. salivarius* ENT-K12, respectively, or *S. salivarius* K12 and *S*. *salivarius* ENT-K12, respectively, and a Lactobacillus strain, in particular *S. salivarius* K12 and *S. salivarius* ENT-K12, respectively, and *L. rhamnosus* LGG, or *S. salivarius* K12 and *S*. *salivarius* ENT-K12, respectively, and *L. casei* 431, as well as isomaltulose, magnesium stearate, HMPC and a flavor preferably selected from those mentioned above, most preferably strawberry flavor, and optionally, but preferably a natural sweetener, like stevia. Alternatively, magnesium stearate is substituted with a natural lubricant/anti-caking agent, preferably with an oat fiber blend, preferably comprising oat fibers, microcrystalline cellulose and sunflower oil refined, for example the product CompactCel^{®} F 200.28 LUB, or with a rice extract blend, preferably comprising rice extract, microcrystalline cellulose, and sunflower oil refined, for example the product CompactCel^{®} F clear 290.02 LUB, or a rice extract blend, preferably comprising rice extract, rice hulls, gum arabic and sunflower oil, for example the product Nu-MAG^{®}, or potato starch, gum Arabica, or with rice hulls, like Nu-FLOW^{®}, or CompactCel^{®} FLO, but most preferably with a rice extract blend in the compressed product of the present invention.

Accordingly, in one embodiment, the compressed product of the present invention comprises any one of the probiotic bacteria or combinations of probiotic bacteria listed above, preferably *S. salivarius* K12 and *S. salivarius* ENT-K12, respectively, or *S. salivarius* K12 and *S*. *salivarius* ENT-K12, respectively, and a Lactobacillus strain, in particular *S. salivarius* K12 and *S. salivarius* ENT-K12, respectively, and *L. rhamnosus* LGG, or *S. salivarius* K12 and *S*. *salivarius* ENT-K12, respectively, and *L. casei* 431, as well as isomaltulose, a rice extract blend, preferably comprising rice extract, rice hulls, gum arabic and sunflower oil, for example the product Nu-MAG^{®}, HMPC, and the above-mentioned flavor.

In one embodiment, the compressed product of the present invention comprises any one of the probiotic bacteria or combinations of probiotic bacteria listed above, preferably *S. salivarius* K12 and *S. salivarius* ENT-K12, respectively, or *S. salivarius* K12 and *S. salivarius* ENT-K12, respectively, and a Lactobacillus strain, in particular *S. salivarius* K12 and *S. salivarius* ENT-K12, respectively, and *L. rhamnosus* LGG, or *S. salivarius* K12 and *S. salivarius* ENT-K12, respectively, and *L. casei* 431, as well as isomaltulose, a rice extract blend, preferably comprising rice extract, microcrystalline cellulose and sunflower oil refined, for example the product CompactCel^{®}LUB, HMPC, and the above-mentioned flavor, preferably strawberry flavor, and optionally, but preferably a natural sweetener, like stevia.

In one embodiment, the compressed product of the present invention comprises any one of the probiotic bacteria or combinations of probiotic bacteria listed above, preferably *S. salivarius* K12 and *S. salivarius* ENT-K12, respectively, or *S. salivarius* K12 and *S. salivarius* ENT-K12, respectively, and a Lactobacillus strain, in particular *S. salivarius* K12 and *S. salivarius* ENT-K12, respectively, and *L. rhamnosus* LGG, or *S. salivarius* K12 and *S. salivarius* ENT-K12, respectively, and *L. casei* 431, as well as isomaltulose, an oat fiber blend, preferably comprising oat fiber, microcrystalline cellulose and sunflower oil refined, for example the product CompactCel^{®}LUB, HMPC, and the above-mentioned flavor, preferably strawberry flavor, and optionally, but preferably a natural sweetener, like stevia.

In one embodiment, the compressed product of the present invention comprises any one of the probiotic bacteria or combinations of probiotic bacteria listed above, preferably *S. salivarius* K12 and *S. salivarius* ENT-K12, respectively, or *S. salivarius* K12 and *S. salivarius* ENT-K12, respectively, and a Lactobacillus strain, in particular *S. salivarius* K12 and *S. salivarius* ENT-K12, respectively, and *L. rhamnosus* LGG, or *S. salivarius* K12 and *S. salivarius* ENT-K12, respectively, and *L. casei* 431, as well as isomaltulose, magnesium stearate, a natural binder like a gum fiber blend, preferably NuBind^{®}, and the above-mentioned flavor preferably strawberry flavor, and optionally, but preferably a natural sweetener, like stevia.

In one embodiment, the compressed product of the present invention comprises any one of the probiotic bacteria or combinations of probiotic bacteria listed above, preferably *S. salivarius* K12 and *S. salivarius* ENT-K12, respectively, or *S. salivarius* K12 and *S. salivarius* ENT-K12, respectively, and a Lactobacillus strain, in particular *S. salivarius* K12 and *S. salivarius* ENT-K12, respectively, and *L. rhamnosus* LGG, or *S. salivarius* K12 and *S. salivarius* ENT-K12, respectively, and *L. casei* 431, as well as isomaltulose, magnesium stearate, a natural binder like a gum fiber blend, preferably CompactCel^{®}DIS, and the above-mentioned flavor, preferably strawberry flavor, and optionally, but preferably a natural sweetener, like stevia.

In one embodiment, the compressed product of the present invention comprises any one of the probiotic bacteria or combinations of probiotic bacteria listed above, preferably *S. salivarius* K12 and *S. salivarius* ENT-K12, respectively, or *S. salivarius* K12 and *S. salivarius* ENT-K12, respectively, and a Lactobacillus strain, in particular *S. salivarius* K12 and *S. salivarius* ENT-K12, respectively, and *L. rhamnosus* LGG, or *S. salivarius* K12 and *S. salivarius* ENT-K12, respectively, and *L. casei* 431, as well as isomaltulose, magnesium stearate, a natural binder like, preferably pregelatinized corn starch, and the above-mentioned flavor, preferably strawberry flavor, and optionally, but preferably a natural sweetener, like stevia.

In one embodiment, the compressed product of the present invention comprises any one of the probiotic bacteria or combinations of probiotic bacteria listed above, preferably *S. salivarius* K12 and *S. salivarius* ENT-K12, respectively, or *S. salivarius* K12 and *S. salivarius* ENT-K12, respectively, and a Lactobacillus strain, in particular *S. salivarius* K12 and *S. salivarius* ENT-K12, respectively, and *L. rhamnosus* LGG, or *S. salivarius* K12 and *S. salivarius* ENT-K12, respectively, and *L. casei* 431, as well as isomaltulose, a rice extract blend, preferably comprising rice extract, rice hulls, gum arabic and sunflower oil, for example the product Nu-MAG^{®}, a natural binder like a gum fiber blend, preferably NuBind^{®}, and the above-mentioned flavor, preferably strawberry flavor, and optionally, but preferably a natural sweetener, like stevia.

In one embodiment, the compressed product of the present invention comprises any one of the probiotic bacteria or combinations of probiotic bacteria listed above, preferably *S. salivarius* K12 and *S. salivarius* ENT-K12, respectively, or *S. salivarius* K12 and *S. salivarius* ENT-K12, respectively, and a Lactobacillus strain, in particular *S. salivarius* K12 and *S. salivarius* ENT-K12, respectively, and *L. rhamnosus* LGG, or *S. salivarius* K12 and *S. salivarius* ENT-K12, respectively, and *L. casei* 431, as well as isomaltulose, a rice extract blend, preferably comprising rice extract, microcrystalline cellulose and sunflower oil refined, for example the product CompactCel^{®}LUB, a natural binder like a gum fiber blend, preferably NuBind^{®}, and the above-mentioned flavor, preferably strawberry flavor, and optionally, but preferably a natural sweetener, like stevia.

In one embodiment, the compressed product of the present invention comprises any one of the probiotic bacteria or combinations of probiotic bacteria listed above, preferably *S. salivarius* K12 and *S. salivarius* ENT-K12, respectively, or *S. salivarius* K12 and *S. salivarius* ENT-K12, respectively, and a Lactobacillus strain, in particular *S. salivarius* K12 and *S. salivarius* ENT-K12, respectively, and *L. rhamnosus* LGG, or *S. salivarius* K12 and *S. salivarius* ENT-K12, respectively, and *L. casei* 431, as well as isomaltulose, an oat fiber blend, preferably comprising oat fiber, microcrystalline cellulose and sunflower oil refined, for example the product CompactCel^{®}LUB, a natural binder like a gum fiber blend, preferably NuBind^{®}, and the above-mentioned flavor, preferably strawberry flavor, and optionally, but preferably a natural sweetener, like stevia.

In one embodiment, the compressed product of the present invention comprises any one of the probiotic bacteria or combinations of probiotic bacteria listed above, preferably *S. salivarius* K12 and *S. salivarius* ENT-K12, respectively, or *S. salivarius* K12 and *S. salivarius* ENT-K12, respectively, and a Lactobacillus strain, in particular *S. salivarius* K12 and *S. salivarius* ENT-K12, respectively, and *L. rhamnosus* LGG, or *S. salivarius* K12 and *S. salivarius* ENT-K12, respectively, and *L. casei* 431, as well as isomaltulose, a rice extract blend, preferably comprising rice extract, rice hulls, gum arabic and sunflower oil, for example the product Nu-MAG^{®}, a natural binder like a gum fiber blend, preferably CompactCel^{®}DIS, and the above-mentioned flavor, preferably strawberry flavor, and optionally, but preferably a natural sweetener, like stevia.

In one embodiment, the compressed product of the present invention comprises any one of the probiotic bacteria or combinations of probiotic bacteria listed above, preferably *S. salivarius* K12 and *S. salivarius* ENT-K12, respectively, or *S. salivarius* K12 and *S. salivarius* ENT-K12, respectively, and a Lactobacillus strain, in particular *S. salivarius* K12 and *S. salivarius* ENT-K12, respectively, and *L. rhamnosus* LGG, or *S. salivarius* K12 and *S. salivarius* ENT-K12, respectively, and *L. casei* 431, as well as isomaltulose, a rice extract blend, preferably comprising rice extract, microcrystalline cellulose and sunflower oil refined, for example the product CompactCel^{®}LUB, a natural binder like a gum fiber blend, preferably CompactCel^{®}DIS, and the above-mentioned flavor, preferably strawberry flavor, and optionally, but preferably a natural sweetener, like stevia.

In one embodiment, the compressed product of the present invention comprises any one of the probiotic bacteria or combinations of probiotic bacteria listed above, preferably *S. salivarius* K12 and *S. salivarius* ENT-K12, respectively, or *S. salivarius* K12 and *S. salivarius* ENT-K12, respectively, and a Lactobacillus strain, in particular *S. salivarius* K12 and *S. salivarius* ENT-K12, respectively, and *L. rhamnosus* LGG, or *S. salivarius* K12 and *S. salivarius* ENT-K12, respectively, and *L. casei* 431, as well as isomaltulose, an oat fiber blend, preferably comprising oat fiber, microcrystalline cellulose and sunflower oil refined, for example the product CompactCel^{®}LUB, a natural binder like a gum fiber blend, preferably CompactCel^{®}DIS, and the above-mentioned flavor, preferably strawberry flavor, and optionally, but preferably a natural sweetener, like stevia.

In one embodiment, the compressed product of the present invention comprises any one of the probiotic bacteria or combinations of probiotic bacteria listed above, preferably *S. salivarius* K12 and *S. salivarius* ENT-K12, respectively, or *S. salivarius* K12 and *S. salivarius* ENT-K12, respectively, and a Lactobacillus strain, in particular *S. salivarius* K12 and *S. salivarius* ENT-K12, respectively, and *L. rhamnosus* LGG, or *S. salivarius* K12 and *S. salivarius* ENT-K12, respectively, and *L. casei* 431, as well as isomaltulose, a rice extract blend, preferably comprising rice extract, rice hulls, gum arabic and sunflower oil, for example the product Nu-MAG^{®®}, a natural binder, preferably pregelatinized corn starch, and the above-mentioned flavor, preferably strawberry flavor, and optionally, but preferably a natural sweetener, like stevia.

In one embodiment, the e compressed product of the present invention comprises any one of the probiotic bacteria or combinations of probiotic bacteria listed above, preferably *S. salivarius* K12 and *S. salivarius* ENT-K12, respectively, or *S. salivarius* K12 and *S. salivarius* ENT-K12, respectively, and a Lactobacillus strain, in particular *S. salivarius* K12 and *S. salivarius* ENT-K12, respectively, and *L. rhamnosus* LGG, or *S. salivarius* K12 and *S. salivarius* ENT-K12, respectively, and *L. casei* 431, as well as isomaltulose, a rice extract blend, preferably comprising rice extract, microcrystalline cellulose and sunflower oil refined, for example the product CompactCel^{®}LUB, a natural binder, preferably pregelatinized corn starch, and the above-mentioned flavor, preferably strawberry flavor, and optionally, but preferably a natural sweetener, like stevia.

In one embodiment, the compressed product of the present invention comprises any one of the probiotic bacteria or combinations of probiotic bacteria listed above, preferably *S. salivarius* K12 and *S. salivarius* ENT-K12, respectively, or *S. salivarius* K12 and *S. salivarius* ENT-K12, respectively, and a Lactobacillus strain, in particular *S. salivarius* K12 and *S. salivarius* ENT-K12, respectively, and *L. rhamnosus* LGG, or *S. salivarius* K12 and *S. salivarius* ENT-K12, respectively, and *L. casei* 431, as well as isomaltulose, an oat fiber blend, preferably comprising oat fiber, microcrystalline cellulose and sunflower oil refined, for example the product CompactCel^{®}LUB, a natural binder, preferably pregelatinized corn starch, and the above-mentioned flavor, preferably strawberry flavor, and optionally, but preferably a natural sweetener, like stevia.

In one embodiment, the compressed product of the present invention comprises any one of the probiotic bacteria or combinations of probiotic bacteria listed above, preferably *S. salivarius* K12 and *S. salivarius* ENT-K12, respectively, or *S. salivarius* K12 and *S. salivarius* ENT-K12, respectively, and a Lactobacillus strain, in particular *S. salivarius* K12 and *S. salivarius* ENT-K12, respectively, and *L. rhamnosus* LGG, or *S. salivarius* K12 and *S. salivarius* ENT-K12, respectively, and *L. casei* 431, as well as isomaltulose, a rice extract blend, preferably comprising rice extract, rice hulls, gum arabic and sunflower oil, for example the product Nu-MAG^{®}, a natural binder, preferably pregelatinized corn starch, and the above-mentioned flavor, preferably strawberry flavor, and optionally, but preferably a natural sweetener, like stevia.

In one embodiment, the compressed product of the present invention comprises any one of the probiotic bacteria or combinations of probiotic bacteria listed above, preferably *S. salivarius* K12 and *S. salivarius* ENT-K12, respectively, or *S. salivarius* K12 and *S. salivarius* ENT-K12, respectively, and a Lactobacillus strain, in particular *S. salivarius* K12 and *S. salivarius* ENT-K12, respectively, and *L. rhamnosus* LGG, or *S. salivarius* K12 and *S. salivarius* ENT-K12, respectively, and *L. casei* 431, as well as isomaltulose, a rice extract blend, preferably comprising rice extract, microcrystalline cellulose and sunflower oil refined, for example the product CompactCel^{®}LUB, a natural binder, preferably pregelatinized corn starch, and the above-mentioned flavor, preferably strawberry flavor, and optionally, but preferably a natural sweetener, like stevia.

In one embodiment, the compressed product of the present invention comprises any one of the probiotic bacteria or combinations of probiotic bacteria listed above, preferably *S. salivarius* K12 and *S. salivarius* ENT-K12, respectively, or *S. salivarius* K12 and *S. salivarius* ENT-K12, respectively, and a Lactobacillus strain, in particular *S. salivarius* K12 and *S. salivarius* ENT-K12, respectively, and *L. rhamnosus* LGG, or *S. salivarius* K12 and *S. salivarius* ENT-K12, respectively, and *L. casei* 431, as well as isomaltulose, an oat fiber blend, preferably comprising oat fiber, microcrystalline cellulose and sunflower oil refined, for example the product CompactCel^{®}LUB, a natural binder, preferably pregelatinized corn starch, and the above-mentioned flavor, preferably strawberry flavor, and optionally, but preferably a natural sweetener, like stevia.

Nu-MAG^{®}, Nu-FLOW^{®}, NuBind^{®}, CompactCel^{®}LUB, CompactCel^{®}FLO, and CompactCel^{®}DIS are natural excipients, *i.e.,* natural lubricants, anti-caking agents, and binders, respectively. They are composed of natural organic ingredients and do not comprise for example silica or magnesium stearate and are thus suitable for "clean label" products. For example, Nu-MAG^{®} is a rice extract blend and comprises rice extract, rice hulls, gum arabic and sunflower oil. Nu-FLOW^{®} is a rice concentrate (a concentrate of silica from rice) and comprises rice hulls. NuBind^{®} is a gum fiber blend and comprises guar gum, gum arabic, agave fiber, rice hulls, and agave syrup. CompactCel^{®}LUB is available in two variants, *i.e.,* either comprising rice extract, microcrystalline cellulose and sunflower oil refined, or oat fiber, microcrystalline cellulose and sunflower oil refined.

Optionally, one or more further active ingredients can be used, which are for example those vitamins and minerals mentioned above. In a preferred embodiment, vitamin D3 (cholecalciferol) is used as further active ingredient. Vitamin D3 can be formulated into the product in form of a vitamin D3 premix, which comprises 2.5 µg/mg vitamin D3. In one embodiment, the product of the present invention comprises 4 to 10 µg vitamin D3, *i.e.* cholecalciferol. In particular, the compressed product preferably comprises 5 to 10 µg vitamin D3.

The contents of the excipient(s) and active ingredient(s) may vary and a person skilled in the art knows how to properly mix the ingredients in order to produce a product which has the desired properties, *i.e.* the properties of the compressed product as described above regarding CFU amount, water activity, disintegration time, average mass, resistance to crushing, and/or friability.

Products of the present invention weight at least enough to formulate 5 mg of the probiotically active organisms, preferably to formulate at least 30 mg of the probiotic microorganism and only weight so much that it is still convenient to administer, *i.e.* to suck the compressed product. The weight of the product can be from about 100 mg to about 2000 mg, wherein the weight of the compressed product ranges preferably from about 500 mg to about 1000 mg. Preferably, the compressed product of the invention weighs about 800 mg ± 10 mg.

The isomaltulose comprises at least 10% and up to 97% of the product of the present invention. Preferably, the amount of isomaltulose is between 50% and 97%, more preferably between 58% and 96%

In one embodiment, the compressed product of the present invention comprises at least 10% and up to 97% isomaltulose. Preferably, the amount of isomaltulose is between 80% and 97%, more preferably between 82% and 92%, more preferably 82.8%, 83.2%, 85.3%, 85.7%, 90.2 %, 90.6 %, 91.4 %, 91.5 %, or 91.9 % of the compressed product. Assuming that the compressed product of the present invention weights 810 mg, the compressed product comprises between 81 mg and 786 mg isomaltulose, preferably between 648 mg and 786 mg, more preferably between 664 mg and 745 mg, more preferably about 670 mg, 674 mg, 691 mg, 694 mg, 731 mg, 734 mg, 740 mg, 741 mg or 744 mg isomaltulose.

The probiotic microorganisms comprise at least 0.3% and up to 20% of the product of the present invention. The product of the present invention preferably comprises between 5 mg to 160 mg probiotic bacteria, more preferably between 30 mg and 120 mg, most preferably 40 mg, 50 mg, 60 mg, 100 mg or 120 mg.

The probiotic microorganisms comprise at least 0.6% and up to 20% of the compressed product of the present invention. Preferably, the content of the probiotic microorganisms is between 3% and 15%, more preferably about 6.2%, 7.4%, 12.3% or 14.8%. The compressed product comprises between 5 mg to 160 mg probiotic bacteria, preferably between 30 mg and 120 mg, more preferably 40 mg, 50 mg, 60 mg, 100 mg or 120 mg.

For example, the compressed product of the present invention, comprises 100 mg or 120 mg of *Lactobacillus paracasei* in total, either 100 mg or 120 mg of each of the strains LP-33 or GMNL-133 alone or 100 mg or 120 mg of both strains in combination; or 50 mg of *Streptococcus salivarius* K12 and *S. salivarius* ENT-K12, respectively; or 40 mg of *Streptococcus salivarius* M18; or 30 mg of *Streptococcus salivarius* K12 and *S. salivarius* ENT-K12, respectively, and 30 mg of *Lactobacillus rhamnosus* LGG; or 30 mg of *Streptococcus salivarius* K12 and *S. salivarius* ENT-K12, respectively, and 30 mg of *Lactobacillus casei* 431; or 30 mg of *Streptococcus salivarius* K12 and *S. salivarius* ENT-K12, respectively, and 30 mg of *Bifidobacterium animalis subsp. lactis* BB-12; or 20 mg of *Streptococcus salivarius* K12 and *S. salivarius* ENT-K12, respectively, 20 mg of *Lactobacillus rhamnosus* LGG and 20 mg of *Bifidobacterium animalis subsp. lactis* BB-12; or 20 mg of *Streptococcus salivarius* K12 and *S. salivarius* ENT-K12, respectively, 20 mg of *Lactobacillus casei* and 20 mg of *Bifidobacterium animalis subsp. lactis* BB-12; or 50 mg of *Streptococcus salivarius* K12 and *S. salivarius* ENT-K12, respectively, and 50 mg of *Lactobacillus paracasei* LP-33; or 50 mg of *Streptococcus salivarius* K12 and *S. salivarius* ENT-K12, respectively, and 50 mg of *Lactobacillus paracasei* GMNL-133.

It will be appreciated that useful probiotically active organisms can be of a genetically modified strain of one of the above organisms. The term "genetically modified" as used herein indicates any modification of DNA sequences coding for genes and modifications of sequences that regulate the expression of genes. Accordingly, genetic modification can be based on construction or selection of mutants of microorganism or it can be based on recombinant DNA technology.

As used herein the term "mutant" is comprised by the conventional meaning of that term, *i.e.* it refers to strains obtained by subjecting a microbial strain to any conventionally used mutagenization treatment including treatment with a chemical mutagen such as ethanemethane sulphonate (EMS) or N-methyl-N'-nitro-N-nitroguanidine (NTG), UV light or to spontaneously occurring mutants which are selected on the basis of a desired characteristic such as *e.g.* antibiotic and/or gastric acid-resistance. It is also possible to select useful genetically modified organisms according to the invention by random mutagenesis or by selection of spontaneously occurring mutants, *i.e.* without the use of recombinant DNA technology. Mutants of the above-mentioned organisms also can be provided by recombinant DNA technology including sitedirected mutagenesis, PCR techniques and other *in vitro* or *in vivo* modifications and insertion of DNA sequences.

The product of the present invention preferably comprises 10⁵, 10⁶, 10⁹ or even 10¹¹ CFU per g. In a preferred embodiment, the product of the present invention comprises between 5 x 10⁹ and 10 x 10⁹ CFUs of the probiotic microorganisms. This will be obtained by using about 40 mg to 120 mg of the probiotic strains when producing the product of the present invention.

As regards the further excipients and active ingredient, the compressed product of the present invention comprises in a preferred embodiment 1 % (about 8 mg) magnesium stearate and its natural substitute as defined above, for example a rice extract blend, preferably CompactCel^{®}LUB, respectively, 1 % (about 8 mg) aromatizing agent, preferably strawberry flavor, and 0.4% of a vitamin and/or mineral, preferably 5-10 µg vitamin D3 (cholecalciferol), which can be provided for example in a form of a premix (0.4 %) which comprises 2.5 µg/mg vitamin D3. Said compressed product is preferably produced by dry compression.

As regards the further excipients and active ingredient, the compressed product of the present invention comprises in another preferred embodiment 1 % (about 8 mg) magnesium stearate and its natural substitute as defined above, for example a rice extract blend, preferably CompactCel^{®}LUB, respectively, 1 % (about 8 mg) aromatizing agent, preferably strawberry flavor, 0.1 % (about 0.74 mg) HMPC, and 0.4% of a vitamin and/or mineral, preferably 5-10 µg vitamin D3 (cholecalciferol), which can be provided for example in a form of a premix (0.4 %) which comprises 2.5 µg/mg vitamin D3. Said compressed product is preferably produced by compression including a wet granulation step.

As regards the further excipients and active ingredient, the compressed product of the present invention comprises in another preferred embodiment 1 % (about 8 mg) magnesium stearate and preferably its natural substitute as defined above, for example a rice extract blend, preferably CompactCel^{®}LUB, respectively, 1 % (about 8 mg) aromatizing agent, preferably strawberry flavor, 0.9 % (about 7.42 mg) pregelatinized corn starch, 0.04 % (about 0.32 mg) of a natural sweetener, preferably stevia, and 0.25% of a vitamin and/or mineral, preferably 5-10 µg vitamin D3 (cholecalciferol), which can be provided for example in a form of a premix (0.4 %) which comprises 2.5 µg/mg vitamin D3. Said compressed product is preferably produced by compression including a wet granulation step. An exemplarily compressed product comprises the ingredients as shown in Tables 2A, 2B, and 2C.

**Table 2A: Ingredients of an exemplarity isomaltulose/probiotics tablet.**

| | **Function** | **Amount (mg)** | **Percentage (%)** |
|---|---|---|---|
| *Streptococcus salivarius* K12/ENT-K12 | Active ingredient / probiotic microorganism | 50.0 | 6.17 |
| Vitamin D3 premix | Active ingredient | 3.2 | 0.40 |
| Isomaltulose | Bulking agent | 740.8 | 91.45 |
| Magnesium stearate (vegetable) | Anticaking agent, lubricant | 8.0 | 0.99 |
| Strawberry flavor | Flavor | 8.0 | 0.99 |
| **Total** | | **810** | **100** |

**Table 2B: Ingredients of an exemplarity isomaltulose/probiotics tablet.**

| | **Function** | **Amount (mg)** | **Percentage (%)** |
|---|---|---|---|
| *Streptococcus salivarius* K12/ENT-K12 | Active ingredient / probiotic microorganism | 50.0 | 6.17 |
| Vitamin D3 premix | Active ingredient | 3.2 | 0.40 |
| Isomaltulose | Bulking agent | 740.06 | 91.36 |
| Hydroxypropylmethlycellulose | Binder | 0.74 | 0.09 |
| Magnesium stearate (vegetable) | Anticaking agent, lubricant | 8.0 | 0.99 |
| Strawberry flavor | Flavor | 8.0 | 0.99 |
| **Total** | | **810** | **100** |

**Table 2C: Ingredients of an exemplarity isomaltulose/probiotics tablet.**

| | **Function** | **Amount (mg)** | **Percentage (%)** |
|---|---|---|---|
| *Streptococcus salivarius* K12/ENT-K12 | Active ingredient / probiotic microorganism | 50.0 | 6.17 |
| Vitamin D3 premix | Active ingredient | 2.0 | 0.24 |
| Isomaltulose | Bulking agent | 734.16 | 90.64 |
| Pregelatinized corn starch | Binder | 7.42 | 0.92 |
| CompactCel^{®} LUB | Anticaking agent, lubricant | 8.1 | 0.99 |
| Strawberry flavor | Flavor | 8.0 | 0.99 |
| Stevia | Natural sweetener | 0.32 | 0.04 |
| **Total** | | **810** | **100** |

Of course, magnesium stearate can be substituted with a natural lubricant/anti-caking agent like a rice extract blend, for example the product Nu-MAG^{®}, or the product CompactCel^{®} LUB (CompactCel^{®} F clear 290.02 LUB, Biogrund GmbH, Huenstetten, Germany), an oat fiber blend, for example the product CompactCel^{®} LUB (CompactCel^{®} F 200.28 LUB, Biogrund GmbH, Huenstetten, Germany) or further products, like potato starch, or gum Arabica, powdered cellulose, like JELUCEL^{®}, native potato starch, inulin, rice fibers, or rice hulls, for example Nu-FLOW^{®}, or CompactCel^{®}FLO, but preferably with a rice extract blend, preferably with CompactCel^{®} LUB, and/or HMPC can be substituted with a natural binder like CompactCel^{®}DIS, NuBind^{®}, or preferably pregelatinized corn starch. Furthermore, CompactCel^{®} LUB and/or pregelatinized corn starch can be substituted with other natural lubricants/binders, like those mentioined above.

The compressed product of the present invention may also comprise silicon dioxide, but this can also be substituted with a natural anti-caking agent like rice hulls, for example CompactCel^{®}FLO, or Nu-FLOW^{®}. Preferably, the compressed product does not comprise magnesium stearate and silicon dioxide, but instead a rice extract blend.

In case more of the probiotic microorganisms are comprised in the compressed product, the amount of isomaltulose will be reduced so that the compressed product still weights about 810 mg. In case less of the probiotic microorganisms are comprised in the compressed product, the amount of isomaltulose will be increased so that the compressed product still weights about 810 mg. In case the compressed product of the present invention comprises more or less vitamin D3, or any other of the vitamins and/or minerals mentioned above, or in case vitamin D3 (or any other of the above-mentioned vitamins and/or minerals) is completely omitted, the amount of isomaltulose will be adapted accordingly.

In a most preferred embodiment, the compressed product of the present invention has the ingredients as listed in Table 2C.

The present invention further relates to a package comprising the compressed product of the present invention, for example a container, *i.e.* bottle or a blister pack.

As mentioned above, probiotics are defined as live microorganisms that, when administered in adequate amounts, confer a health benefit on the host. The beneficial effects of probiotics may be mediated by a direct antagonistic effect against specific groups of undesired organisms, resulting in a decrease of their numbers, by an effect on the metabolism of such groups of organisms or by a general stimulatory effect on the immune system of animal or human hosts. Thus, the present invention relates to the use of the product for reducing the occurrence of infections with pathogens for example in the oral cavity, the respiratory tract, the gastrointestinal tract and the vagina or of the skin. Accordingly, the product of the present invention is suitable for supporting healthy mouth microflora, healthy upper and lower respiratory tract microflora, healthy skin, improving weight management, maintaining healthy gut microflora, healthy vaginal flora; maintaining a normal digestion or supporting healthy gut mobility, bowel movement and/or healthy stool frequency, stool consistency and/or form in a subject.

In the present context, the expression "reducing the occurrence of infections" indicates that the above-mentioned infections or symptoms caused by the presence of pathogens occurs at a reduced frequency or seriousness as compared to a human or animal subject who or which is not being treated with the compressed product of the present invention. In the present context treatment is also to be construed as encompassing prevention or prophylaxis in addition to cure.

It is furthermore documented that probiotic microorganisms produce essential vitamins and nutrients required by the intestinal cells and furthermore assist with degradation of certain nutrients and even activate cell-mediated immune effector functions. Thus probiotic microorganisms can improve the general health status of a mammal.

A number of reports indicate that intake of probiotic microorganisms may not only reduce the occurrence of infections and are health promoting, but indeed contribute to the treatment of disease; see for example Wescombe et al., Future Microbiol. (2012) 7(12), 1355-1371; Zupancic et al., Probiotics Antimicrob Proteins (2017) 9(2), 102-110; Wilcox et al., Clin Microbiol Infect (2019) 25(6), 673-680; Marom et al., Medicine (Baltimore) (2016) 95(6), e2695; Clark, Curr Opin Immunol (2020) 66, 42-49; Bertuccioli et al., Nutrafoods (2019) 2, 80-88. Thus, the present invention relates to a product for use in the treatment of various diseases or conditions like otitis, preferably otitis media; upper respiratory tract infections, preferably tonsillitis or pharyngitis; lower respiratory tract infections, preferably bronchitis or pneumonia; diseases and inflammations of the oral cavity, preferably caries, oral mucositis, periodontitis, candidiasis, and/or oral lichen planus; halitosis; skin disorders, preferably acne and/or dermatitis; gastro-intestinal problems like diarrhea, gastroenteritis, irritable bowel syndrome (IBS), inflammatory bowel disease (IBD), urogenital infection; allergies, lactose intolerance or immune diseases preferably allergic rhinitis, or mastitis in a subject.

These health benefitting effects have been verified is various studies and described for example in Fijan (2014), Int J Environ Res Public Health. 11(5): 4745-4767; EP 1 483 366 B1; EP 2 581 461 B1; US 6,994,848 B2; WO 2005/007178 A1; Di Pierro et al., Drug Healthc Patient Saf. 6 (2014), 15-20.

The term "individual" or "subject" as used herein includes humans, horses, dogs, cats, pigs, sheep, cattle, goats but is not limited thereto. Preferably, the individual is a human. The product of the present invention can be administered to the individual at any age, *e.g.* childhood, adolescence, or adulthood.

In general, the amount of probiotics administered via the product of the present invention to the individual will be an amount of an active agent high enough to deliver the desired benefit, but low enough to avoid serious side effects. Specific dosages can vary widely according to various individual variables including size, weight, age, disease severity and responsiveness to therapy. Methods for determining the appropriate dosage may be determined by the consumer as they deem appropriate, or on a case-by-case basis by an attending a pharmacist or clinician. Such determinations are routine to one of ordinary skill in the art (see for example, Remington's Pharmaceutical Sciences, 8th ed., Gennaro, ed., Mack Publishing Company, Easton, Pa., 1990).

The product of the present invention may need to be administered to the patient once only or more usually repeatedly. Repeat prophylactic or therapeutic treatments may be once a month, once a week, once a day, twice a day, three times a day, four times a day, five times a day, six times a day or as may otherwise be required.

The present invention also relates to a method of producing a product which comprises at least probiotic bacteria as active ingredient and isomaltulose as excipient. The ingredients and features of the product to be produced with the method of the present invention are those as defined hereinbefore.

Methods of pressing tablets are known to the persons skilled in the art and thus, only the most important steps are described in the following.

The probiotic bacteria which are used in the method of the present invention can be provided in several forms, but preferably they are provided freeze-dried.

The method of the present invention comprises at least mixing the probiotic microorganisms with isomaltulose and compressing the mixture to a compressed product with a compression pressure of 6 to 10 kN/compressed product. The method of the present invention further comprises blending/homogenizing the mixture before compressing the mixture to the compressed product. This is performed to achieve blend uniformity and to uniformly distribute all ingredients. The optimum mixing time and speed to achieve a homogenous mixture can be evaluated by the person skilled in the art. For mixing and blending a fluidized bed granulator can be, and is preferably used.

In one embodiment, the method of the present invention further comprises analyzing the product, *e.g*. analyzing one, two, three, four, five, six, or all seven of the following parameters: CFU/g amount, water activity, disintegration time, average mass, resistance to crushing, appearance, and friability of the product.

In a preferred embodiment, the method for producing the compressed product of the present invention comprises the mixing of the probiotic bacteria with isomaltulose and the one or more further excipients as defined hereinbefore, in particular with a lubricant as defined above or with a lubricant and a binder as defined above, preferably wherein the lubricant is magnesium stearate, more preferably a natural substitute like a rice extract blend as defined above, and the binder is HMPC, more preferably a natural substitute like pregelatinized starch.

The lubricant magnesium stearate can also be substituted with a natural lubricant as defined hereinbefore and the anti-caking agent can be substituted with a natural anti-caking agent or ticalcium phosphate as defined hereinbefore.

In one embodiment, the method of the present invention further comprises the mixing of the above-mentioned ingredients with an aromatizing agent as mentioned before, like strawberry flavor, and optionally with a natural sweetener, preferably stevia. Optionally, a further active ingredient, preferably a vitamin and/or mineral salt, most preferably vitamin D3 is also added and mixed with the probiotics (for example in form of a premix), the isomaltulose and the further excipient(s). Preferably, segment mixing is performed wherein the probiotic microorganisms and optionally the further active ingredient(s) are mixed stepwise with parts of the excipients.

The mixed and preferably blended powder is further used for manufacturing the compressed product of the present invention, *i.e.* the blended/homogenized powder is pressed. The compression force is a critical parameter since many probiotically active organisms, including the most interesting probiotic lactic bacteria, are highly sensitive to the pressure caused when formed into tablets by direct compression. Thus, a compression force has to be chosen which allows survival of the probiotics but which leads to a tablet with the desired properties, *i.e.* the properties as described above regarding water activity, disintegration time, average mass, resistance to crushing, CFU count and/or friability.

As a general rule: the lower the compression force the higher the survival rate. However, a low compression force results in less coherent tablets, but the use of isomaltulose results in a tablet with a good coherency and a high survival rate of the probiotic organisms as for example compared to tablets in which isomalt has been used; see the Examples. The compression force to be applied is between to 6 to 10 kN/compressed product or kN/cm², respectively and preferably around 7 to 8 kN/compressed product or kN/cm², respectively.

Also the dwell-time, *i.e.* the time during which the compression is maximal, needs to be considered. According to a preferred embodiment the compression time, *i.e.* the short period of time wherein the compressible formulation is compressed in the tablet press, is less than 1 s, preferably the time is less than 0.5 s, more preferably less than 0.1 s and most preferably less than 0.08 s.

Another point is the heat development during compression. Since probiotics are heat sensitive, the generation of excessive heat during compression should be avoided. Thus, the tablet press is run at a low speed in order to avoid strong heat development. The preferred speed can be tested by a person skilled in the art but preferably the tablet press is run at such speed that about 600 tablets are pressed per minute.

After compression, the compressed product is preferably analyzed regarding its CFU count, disintegration time, its average mass, its resistance to crushing, its friability, its appearance, and/or its water activity. This can be performed by methods known in the art and in particular by the methods as described in the Examples section.

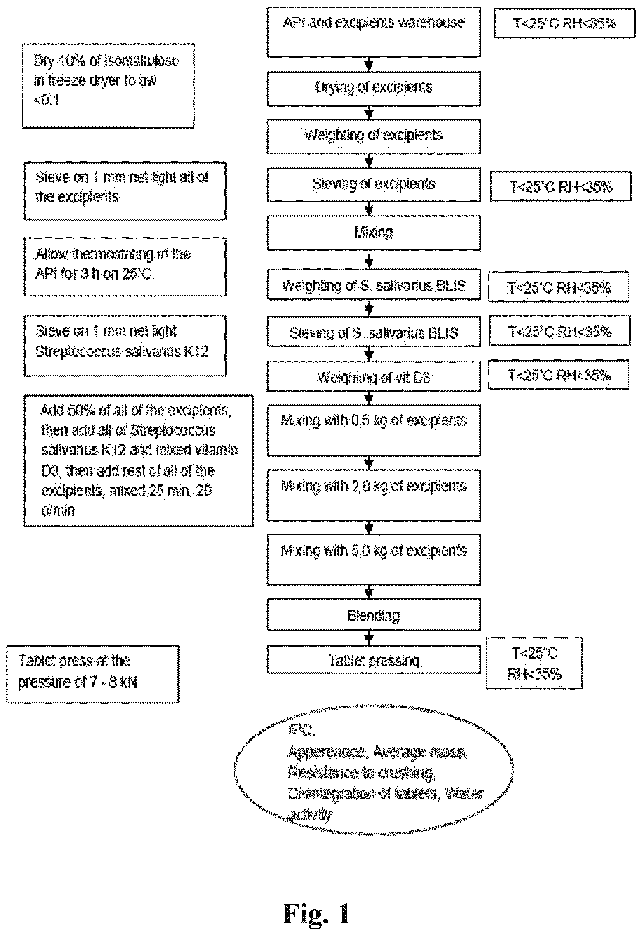

In one embodiment, the method of the present invention does not comprise a granulation step, *i.e.* no slugging and/or roller compaction/ribbon blending is performed, but the blended mixture is directly compressed into the final compressed product. Accordingly, the method of the present invention comprises in one embodiment at least the following steps: (i) mixing the probiotic microorganisms with isomaltulose and with one or more further excipients as defined above, preferably with a lubricant as defined above, preferably with magnesium stearate, and optionally with one or more further active ingredients as defined above, preferably with vitamin D3; (ii) blending the mixture; and (iii) compressing the mixture to a compressed product with a compression force between 5 and 10 kN/compressed product; see also Fig. 1.

Alternatively, the method of producing the compressed product comprises a briquetting step as described in EP 22 168 218.0, wherein the isomaltulose and preferably the other excipients mentioned above are first compressed to briquettes, wherein afterward the briquettes are fractionized and grinded, followed by the addition of the probiotic microorganisms to the grinded briquettes, mixing and preferably blending/homogenizing. Accordingly, the method of the present invention comprises in one embodiment at least the following steps: (i) mixing the isomaltulose with one or more further excipients as defined above, preferably with a lubricant as defined above, preferably with magnesium stearate, and most preferably with a natural substitute, like a rice extract blend as defined above; (ii) compression of the mixed excipients to briquetts; (iii) fractionizing and grinding the briquetts; (iv) addition of the probiotic microorganisms to the grinded briquettes and optionally addition of one or more further active ingredients as defined above, preferably vitamin D3; (v) homogenizing/blending the mixture; and (vi) compressing the mixture to a compressed product, wherein the compression force is between 6 and 10 kN/compressed product. Those steps are in detail described in EP 22 168 218.0.

Alternatively, the method of producing the compressed product comprises a wet granulation step. Wet granulation can in general be performed with water or ethanol as a solvent and using various binders (HPMC, PVP K-25, PVP K-30, PEG 4000, PEG 6000, microcrystalline cellulose, starch, and its derivatives). The wet granulation process is performed in a granulation device by spraying a binder solution on excipients, under controlled pressure conditions (0.15-0.18 MPa) under which the liquid is sprayed. Granulation devices can be for example a highshear granulator, a twin screw granulator or a fluidized bed granulator). During this procedure, wet granules are formed. After the completion of the spraying procedure, the granules are dried at a controlled temperature of 40 ± 2°C, for about 30 minutes. The resulting granulate is cooled to room temperature and water activity and relative humidity are less than 0.25 and less than 1.5%, respectively. With the prepared granulate, the active ingredients are homogenized and tableting is started.

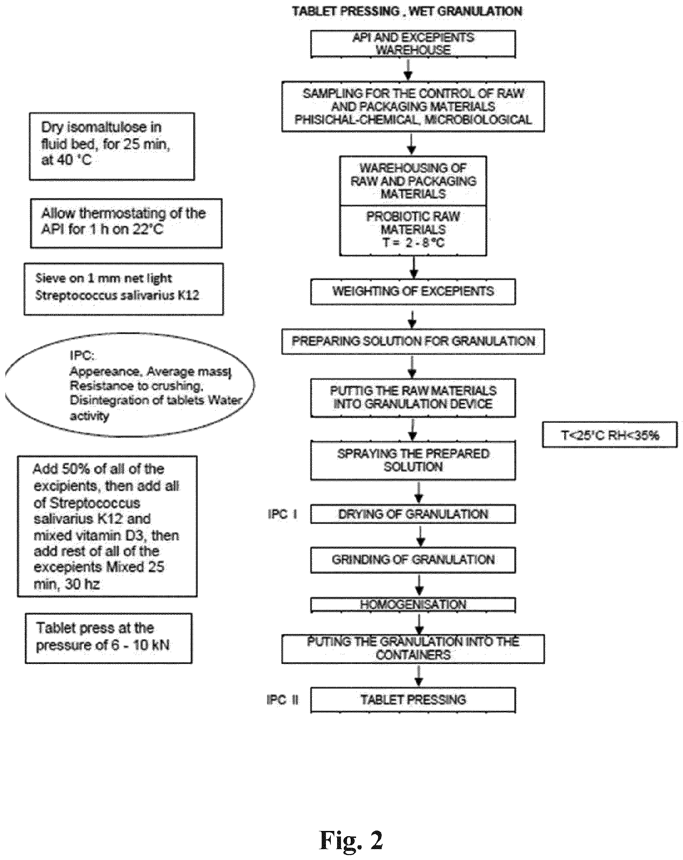

Accordingly, the method of the present invention comprises in one embodiment at least the following steps: (i) mixing the isomaltulose with one or more further excipients as defined above, preferably with a lubricant as defined above, preferably with magnesium stearate, more preferably a natural substitute, prefearbly a rice extract blend as defined above; (ii) spraying a binder solution on the excipients, preferably wherein the binder solution is composed of water and a binder as defined above, preferably wherein the binder is HMPC or pregelatinized corn starch, most preferably pregalatinized corn starch, and preferably wherein the spraying is performed under controlled pressure conditions (0.15-0.18 MPa), thereby forming the wet granules; (iii) drying the granules, preferably at a controlled temperature of 40 ± 2°C, for about 30 minutes, preferably until a water activity and relative humidity of less than 0.25 and less than 1.5%, respectively, is reached; (iv) grinding/milling the granules; (v) addition of the probiotic microorganisms to the grinded granules and optionally addition of one or more further active ingredients as defined above (preferably vitamin D3); (vi) homogenizing/blending the mixture; and (vii) compressing the mixture to a compressed product with a compression force between 6 and 10 kN/compressed product; see also Fig. 2.

The excipients which are used for the preparation of the product of the present invention are preferably dried before further processing. In a preferred embodiment, only 1-10 % of the isomaltulose are dried in a freeze dryer to a water activity of about ≤ 0.1 % and the other 90-99% are not dried; more preferably, only 10 % of the isomaltulose are dried in a freeze dryer to a water activity of about ≤ 0.1 % and the other 90% are not dried (dry compression). Alternatively all of the isomaltulose is dried in a fluid bed for about 25 minutes at 40°C (wet granulation).

As mentioned with regard to the product of the present invention above, the mixture used in the method of the present invention for preparing said product comprises at least 10% and up to 97% isomaltulose. Preferably, the amount of isomaltulose is between 50% and 97%, more preferably between 58% and 96%. The amount of isomaltulose in the mixture for preparing the compressed product is preferably between 80% and 97%, more preferably between 82% and 92%, more preferably 82.8%, 83.2%, 85.3%, 85.7%, 90.2 %, 90.6 %, 91.4%, 91.5 %, or 91.9 %. The probiotic microorganisms will comprise at least 0.3% and up to 20% of the mixture. As regards the compressed product, the probiotics will comprise 0.6% to 20 % of the mixture and preferably, the content of the probiotic microorganisms is between 3% and 15%, more preferably about 6.2%, 7.4%, 12.3% or 14.8%.

As regards the further excipients and active ingredient (next to isomaltulose and the probiotic microorganisms), the mixture used in the method of the present invention to produce the compressed product, in particular by dry compression, comprises in a preferred embodiment 1% of a lubricant/anti-caking agent, preferably magnesium stearate and more preferably its natural substitute, respectively, as defined above, preferably a rice extract blend as defined above like Nu-MAG^{®} or CompactCel^{®}LUB, preferably CompactCel^{®}LUB, and 1 % aromatizing agent, preferably strawberry flavor, and optionally 0.2 % or preferably 0.4 % of vitamin D3 premix (cholecalciferol), which comprises 2.5 µg/mg vitamin D3, and optionally, but preferably 0.04% of a natural sweetener, preferably stevia.

As regards the further excipients and active ingredient (next to isomaltulose and the probiotic microorganisms), the mixture used in the method of the present invention to produce the compressed product, in particular by compression including a wet granulation step, comprises in a preferred embodiment 1 % of a lubricant/anti-caking agent, preferably magnesium stearate and more preferably its natural substitute, respectively, as defined above, preferably a rice extract blend as defined above like Nu-MAG^{®} or CompactCel^{®}LUB, preferably CompactCel^{®}LUB, 1 % aromatizing agent, preferably strawberry flavor, 0.1 % to 0.9% of a binder, preferably 0.1% HMPC or a natural substitute as defined above, preferably CompactCel^{®}DIS, NuBind^{®} or pregelatinized starch, preferably 0.9% pregelatinized corn starch, and optionally 0.2 % or preferably 0.4 % of vitamin D3 premix (cholecalciferol), which comprises 2.5 µg/mg vitamin D3, and optionally, but preferably 0.04% of a natural sweetener, preferably stevia.

Before the mixing and blending steps with the active ingredient(s) are performed, the excipients are weighted, sieved, preferably through a 1 mm net and mixed. Furthermore, the probiotic microorganisms are weighted and sieved, preferably through a 1 mm net and optionally are thermostabilized for 1 to 3 hours before the weighting and sieving step. In case a product in produced which comprises further active ingredients, in particular which comprises vitamin D3, the vitamin D3 premix is also weighted before mixing.

In order to achieve a low water activity of the product which is important for its shelf-life, most of the steps are performed at room temperature and at a relative humidity of about ≤ 35 % as can be derived from Fig. 1 and Fig. 2. Room temperature can be defined as a temperature of ≤ 25°C and more preferably of 15°C to 25°C.

Several documents are cited throughout the text of this specification. However, there is no admission that any document cited is indeed prior art as to the present invention.

A more complete understanding can be obtained by reference to the following specific

### EXAMPLES

### Material and Methods

### Preparation of the compressed product

The preparation of the compressed product is performed according to the schema depicted in Fig. 1 (dry compression) or Fig. 2 (wet granulation) and is exemplarily described for tablets comprising *Streptococcus salivarius* K12, isomaltulose, magnesium stearate, strawberry flavor, and vitamin D3 or for tablets comprising *Streptococcus salivarius* K12, isomaltulose, magnesium stearate, HMPC, strawberry flavor, and vitamin D3. However, the same process is used for tablets of the present invention which are defined hereinbefore.

As regards the dry compression method, the excipients (here: isomaltulose, magnesium stearate, strawberry flavor) are dried, weighted, sieved through a 1 mm net and mixed. In particular, 10 % of the isomaltulose are dried in a freeze-dryer to a water activity of < 0.1, wherein 90 % of the isomaltulose are not dried, and the sieving is performed at room temperature, in particular at < 25 °C, and at a relative humidity of < 35%.

*S. salivarius* K12 is used as freeze-dried product and before further processing, it allowed to thermostate for 3 h at 25°C. Afterwards, *S. salivarius* K12 is weighted and sieved through a 1 mm net, and vitamin D3 is weighted, wherein those steps are performed at room temperature, in particular at < 25 °C, and at a relative humidity of < 35%.

In a next step, 10% of the excipients are mixed with *S. salivarius* K12 and vitamin D3. Afterwards, further 40 % of the excipients are added and mixed and finally the remaining 50 % of the excipients are added and mixed. Mixing is performed for about 25 minutes at 20 o/min. The next step comprises blending and tablet pressing. Tablet pressing is performed at about 7 to 8 kN and the tablet press is run at low speed in order to avoid strong heat production, for example at a speed to produce 600 tablets/min. The tablet pressing step is also performed at room temperature, in particular at < 25 °C, and at a relative humidity of < 35%. The pressed tablets are analyzed with regard to their appearance, average mass, resistance to crushing, disintegration time, friability and/or water activity.

The concentration of the excipients and the active pharmaceutical ingredients in this exemplarily process are as follows:
6.2 % *Streptococcus salivarius* K12,
91.5 % isomaltulose,
1 % magnesium stearate,
1 % strawberry flavor, and
0.4 % vitamin D3 premix.

The vitamin D3 premix can be substituted with isomaltulose and if different amounts of the probiotic microorganism are used, the concentration of isomaltulose is adapted accordingly. As regards the wet granulation compression method, at least the isomaltulose is dried. In particular, the isomaltulose is dried for 25 min at 40°C in a fluid bed. Afterwards, the excipients (here: isomaltulose, magnesium stearate, HMPC, strawberry flavor) are weighted.

*S. salivarius* K12 is used as freeze-dried product and before further processing, it allowed to thermostate for 1 h at 22°C. Afterwards, *S. salivarius* K12 is weighted and sieved through a 1 mm net, and vitamin D3 is weighted.

In a next step, a solution for granulation is prepared, *i.e.* water is mixed with the binder HPMC. Afterwards, the isomaltulose and magnesium stearate are put into the granulation device, *i.e.* isomaltulose and magnesium stearate are mixed. This is followed by spraying the binder solution on the excipients under controlled pressure conditions (0.15-0.18 MPa), thereby forming the wet granules. Afterwards, the granulate is dryed at a controlled temperature of 40 ± 20°C, for about 30 minutes until a water activity and relative humidity of less than 0.25 and less than 1.5%, respectively, is reached. This is followed by cooling and grinding the granulate and by addition of *S. salivarius* K12 and vitamin to the grinded granulate. In the next step, 50% of the excipients are mixed with *S. salivarius* K12 and vitamin D3. Afterwards, the remaining excipients are added and mixed. Mixing is performed for about 25 minutes at 30 hz. The next step comprises tablet pressing. Tablet pressing is performed at about 6 to 10 kN and the tablet press is run at low speed in order to avoid strong heat production, for example at a speed to produce 600 tablets/min. The tablet pressing step is also performed at room temperature, in particular at < 25 °C, and at a relative humidity of < 35%. The pressed tablets are analyzed with regard to their appearance, average mass, resistance to crushing, disintegration time, friability and/or water activity.

The concentration of the excipients and the active pharmaceutical ingredients in this exemplarily process are as follows:
6.2 % *Streptococcus salivarius* K12,
91.4 % isomaltulose,
0.1 % HMPC
1 % magnesium stearate,
1 % strawberry flavor, and
0.4 % vitamin D3 premix.

The vitamin D3 premix can be substituted with isomaltulose and if different amounts of the probiotic microorganism are used, the concentration of isomaltulose is adapted accordingly.

### Determination of colony forming units (CFU)

Determination of CFUs of *S. salivarius, L. casei* and *L. rhamnosis* is performed as described in "Istituto Superiore di Sanità, Metodi microbiologici tradizionali e metodi molecolari per l'analisi degli integratori alimentari a base di o con, probiotici per uso umano, by Paolo Aureli, Alfonsina Fiore, Concetta Scalfaro, Giovanna Franciosa, 2008, ii, 63 p. Rapporti ISTISAN 08/36, ISSN 1123-3117", in particular in chapters 21 and 24.

### Determination of the hardness of the compressed product

The determination of the hardness of the compressed product, *i.e.* its resistance to crushing, is performed as described in chapter 2.9.8 of the European Pharmacopoeia 6.0 by Council of Europe; 6th Edition; published on May 10, 2008; ISBN-10 : 9287160546; ISBN-13 : 978-9287160546.

### Determination of the disintegration time of the compressed product

The determination of the disintegration time of the compressed product is performed as described in chapter 2.9.1 of the European Pharmacopoeia 6.0 by Council of Europe; 6th Edition; published on May 10, 2008; ISBN-10 : 9287160546; ISBN-13 : 978-9287160546.

### Determination of the water activity

The determination of the water activity is performed using the HygroLab 3 of Rotronic AG Bassersdorf, Switzerland, and measurement is performed according to the user manual.

### Determination of the friability of the compressed product

The determination of the friability of the compressed product is performed as described in chapter 2.9.7 of the European Pharmacopoeia 6.0 by Council of Europe; 6th Edition; published on May 10, 2008; ISBN-10 : 9287160546; ISBN-13: 978-9287160546.

### Example 1: Comparison of a lozenge comprising S. salivarius K12 and isomaltulose with a lozenge comprising S. salivarius K12 and isomalt

In a first approach, isomaltulose lozenges comprising about 6.2 % *Streptococcus salivarius* K12, 91.5 % isomaltulose, 1 % magnesium stearate, 1 % strawberry flavor, and 0.4 % vitamin D3 premix have been compared to corresponding isomalt lozenges, which comprise isomalt as bulking agent instead of isomaltulose. In particular, the lozenges comprise 50 mg *S. salivarius* K12 and have been analyzed regarding their hardness, disintegration time, friability, water activity and CFUs of *S. salivarius* K12. The results are shown in Tables 3 and 4.

As can be derived from Tables 3 and 4, the isomaltulose lozenges are not as hard as the isomalt lozenges. However, remarkably the CFUs are in average much higher in the isomaltulose lozenges than in the isomalt lozenges. In particular, although the same concentration of freeze-dried *S. salivarius* K12 has been used as raw material, more viable bacteria are present in the isomaltulose lozenges in comparison to the corresponding lozenges with isomalt. Furthermore, the disintegration time of the isomaltulose lozenges is higher than the disintegration time of the isomalt lozenges. The friability of both lozenges is comparable, but the water activity of the isomalt lozenges is slightly higher.

**Table 3: Characteristics of isomaltulose lozenges comprising 50 mg S. salivarius K12. Two different batches are exemplarily shown (first batch in bold, second batch in italics, wherein values in bold and italics indicate that the values of the first and the second batch are the same).**

| **Time [months]** | **Average mass [mg]** | **Hardness [Newton]** | **Disintegration time [min]** | **Friability [%]** | **Water activity [Aw]** | **Premix CFU/g K12** | **CFU/g K12** |
|---|---|---|---|---|---|---|---|
| 0 | **807** / *946* | ***70-80*** | >***10*** | **1.23** / *1.5* | **0.107** / *0.136* | **1.30E+10** / 1.04E+10 | **1.22E+10** / *9.60E+9* |
| 3 | | | | | **0.166** / *0.174* | | **8.00E+09** / *7.60E+9* |
| 6 | | | | | **0.178** / *0.192* | | **7.03E+09** / *6.90E+9* |
| 9 | | | | | **0.184** / *0.22* | | ***N*/*A*** |
| 12 | | | | | **0.182** / *0.217* | | **3.04E+09** / *4.50E+9* |

**Table 4: Characteristics of isomalt lozenges comprising 50 mg S. salivarius K12. Two different batches are exemplarily shown (first batch in bold, second batch in italics, wherein values in bold and italics indicate that the values of the first and the second batch are the same).**

| **Time [months]** | **Average mass [mg]** | **Hardness [Newton]** | **Disintegration time [min]** | **Friability [%]** | **Water activity [Aw]** | **Premix CFU/g K12** | **CFU/g K12** |
|---|---|---|---|---|---|---|---|
| 0 | **800** / *803* | ***85*** | ***5*** | **1.29** / *1.38* | **0.159** / *0.175* | **7.62E+09 /** 6.52E+09 | **7.08E+09 /** *6.02E+09* |
| 3 | | | | | **0.151** / *0.219* | | **6.58E+09** / *6.02E+09* |
| 6 | | | | | **0.185** / *0.213* | | **6.07E+09** / *5.10E+09* |
| 9 | | | | | **0.19** / *0.205* | | ***N*/*A*** |
| 12 | | | | | **0.171** / *0.206* | | **5.18E+09** / *3.53E+09* |

### Example 2: Comparison of a lozenge comprising S. salivarius K12, a Lactobacillus strain and isomaltulose with a lozenge comprising S. salivarius K12, a Lactobacillus strain and isomalt

In a second approach, isomaltulose lozenges comprising *S. salivarius K12* and *L. rhamnosus* LGG or *S. salivarius K12* and *L. casei* in different concentrations (5%, 6.2%, 7.4%, 9.3%) isomaltulose (92.7%, 91.5%, 88.4%, 85.3%) magnesium stearate (1%), strawberry flavor (1%), and vitamin D3 premix (0.4%) have been compared to corresponding isomalt lozenges, which comprise isomalt as bulking agent instead of isomaltulose. In particular, the lozenges have been analyzed regarding their hardness, disintegration time, friability, water activity and CFUs of *S*. *salivarius* K12 directly after compressing (time point 0). The results are shown in Tables 5 and 6.

**Table 5: Characteristics of isomaltulose lozenges comprising different amounts of S. salivarius K12, L. rhamnosus LGG, and L. casei 431 measured directly after compression (time point 0).**

| **Dose** | **Average mass [mg]** | **Hardness [Newton]** | **Disint. time [min]** | **Friability [%]** | **Water activity [Aw]** | **CFU/g K12** | **CFU/g LGG** | **CFU/g casei** |
|---|---|---|---|---|---|---|---|---|
| K12-50mg; LGG-50mg | 803 | 83 | 11 | 1,25 | 0,129 | 7,03E+09 | 1,05E+10 | |
| K12-25mg; LGG-50mg | 803 | 82 | 10 | 1,24 | 0,14 | 3,13E+09 | 1,33E+10 | |
| K12-30mg; LGG-30mg | 803 | 83 | 10 | 1,25 | 0,149 | 5,08E+09 | 7,09E+09 | |
| K12-30mg; casei-30mg | 803 | 82 | 11 | 1,26 | 0,137 | 4,95E+09 | | 3,25E+09 |
| K12-20mg; LGG-20mg | 803 | 85 | 10 | 1,24 | 0,144 | 3,05E+09 | 3,54E+09 | |

**Table 6: Characteristics of isomalt lozenges comprising different amounts of S. salivarius K12, L. rhamnosus LGG, and L. casei 431 measured directly after compression (time point 0).**

| **Dose** | **Average mass [mg]** | **Hardness [Newton]** | **Disint. time [min]** | **Friability [%]** | **Water activity [Aw]** | **CFU/g K12** | **CFU/g LGG** | **CFU/g casei** |
|---|---|---|---|---|---|---|---|---|
| K12-50mg; LGG-50mg | 804 | 84 | 5 | 1,24 | 0,182 | 7,23E+09 | 8,15E+09 | |
| K12-25mg; LGG-50mg | 803 | 84 | 5 | 1,24 | 0,193 | 3,74E+09 | 1,06E+10 | |
| K12-30mg; LGG-30mg | 803 | 84 | 5 | 1,24 | 0,189 | 4,43E+09 | 7,12E+09 | |
| K12-30mg; L.casei-30mg | 803 | 85 | 5 | 1,24 | 0,191 | 5,10E+09 | | 3,34E+09 |
| K12-20mg; LGG-20mg | 803 | 85 | 5 | 1,25 | 0,195 | 3,78E+09 | 4,58E+09 | |

As can be derived from Tables 5 and 6, the hardness, the CFU amount of the probiotics and the friability of both isomaltulose and isomalt lozenges is similar. The disintegration time of the isomaltulose lozenges is higher than the disintegration time of the isomalt lozenges and the water activity of the isomaltulose lozenges is lower.

## Claims

1. A product which comprises at least probiotic microorganisms as active ingredient and isomaltulose as excipient, wherein the product is a compressed product, wherein the compressed product is a lozenge, and wherein the probiotic microorganisms are mixed with the isomaltulose, wherein the mixture is homogenized, and wherein the mixture is compressed to the compressed product, wherein compression is performed with a pressure of 6 to 10 kN/compressed product.

2. The product of claim 1, wherein the probiotic microorganisms are lactic acid bacteria.

3. The product of claim 1 or 2, wherein the probiotic microorganisms are of the genus *Streptococcus, Lactobacillus, Lactococcus, Enterococcus,* and/or *Bifidobacterium,* preferably wherein the probiotic microorganisms are selected from the group consisting of *Streptococcus salivarius,* preferably *Streptococcus salivarius* K12, *Streptococcus salivarius* ENT-K12, or *Streptococcus salivarius* M18; *Lactobacillus rhamnosus,* preferably *Lactobacillus rhamnosus* LGG; *Lactobacillus casei*; *Lactobacillus paracasei*; *Lactobacillus fermentum*; *Lactobacillus crispatus*; *Lactobacillus plantarum; Bifidobacterium animalis subsp. lactis*; *Lactococcus lactis; Enterococcus faecalis; Lactobacillus reuteri*; either alone or in combination.

4. The product of any one of claims 1 to 3, which further comprises one or more excipients, preferably a lubricant and/or an anti-caking agent, and/or a binder, and/or an aromatizing agent, preferably wherein the lubricant is magnesium stearate or a natural substitute, wherein the anti-caking agent is silicon dioxide, tricalcium phosphate or a natural substitute, wherein the binder is HMPC or a natural substitute, and/or wherein the aromatizing agent is a flavor, preferably strawberry flavor, and optionally wherein the product further comprises a natural sweetener, and/or one or more active ingredients, preferably vitamins, minerals and/or fructooligosaccharides, preferably wherein the vitamin is vitamin D3.

5. The product of any one of claims 1 to 3, wherein the product further comprises
(i) a lubricant/an anti-caking agent, preferably wherein the lubricant/anti-caking agent is magnesium stearate or a natural substitute; an aromatizing agent, preferably wherein the aromatizing agent is a flavor, preferably strawberry flavor; and optionally a natural sweetener and/or one or more active ingredients, preferably a vitamin, preferably vitamin D3; or
(ii) a lubricant/an anti-caking agent, preferably wherein the lubricant/anti-caking agent is magnesium stearate or a natural substitute; a binder, preferably wherein the binder is hydroxypropyl methylcellulose (HMPC) or a natural substitute; an aromatizing agent, preferably wherein the aromatizing agent is a flavor, preferably strawberry flavor; and optionally a natural sweetener, and/or one or more active ingredients, preferably a vitamin, preferably vitamin D3.

6. The product of any one of claims 1 to 3, wherein the product further comprises a rice extract blend as lubricant/anti-caking agent, pregelatinized corn starch as binder, a flavor as aromatizing agent, preferably strawberry flavor, a natural sweetener, preferably stevia, and vitamin D3 as further active ingredient.

7. The product of any one of claims 1 to 6, wherein the content of isomaltulose is at least 80 %, preferably between 80 % and 97 %, more preferably between 82% and 92%, and/or wherein the content of the probiotic microorganisms is between 0.6% and 20%, preferably between 3 % and 15 %; and/or wherein the product comprises between 5 mg and 160 mg, preferably between 30 mg and 120 mg, more preferably 40 mg, 50 mg, 60 mg, 100 mg or 120 mg of the probiotic microorganisms.

8. The product of any one of claims 1 to 7, which has a disintegration time of more than 5 minutes, preferably between 10 and 30 minutes, more preferably between 10 and 11 minutes and/or which has a friability of about 1.2 to 1.5 %, preferably of about 1.25 %, wherein the product comprises at least 80 %, preferably between 80 % and 97 %, more preferably between 82% and 92% isomaltulose and further comprises
(i) a lubricant/an anti-caking agent; an aromatizing agent; and optionally a natural sweetener; and/or one or more active ingredients; or
(ii) a lubricant/an anti-caking agent; a binder; an aromatizing agent; and optionally a natural sweetener; and/or one or more active ingredients.

9. Use of isomaltulose as excipient in a product as defined in any one of claims 1 to 8.

10. A package comprising the product of any one of claims 1 to 8.

11. The product of any one of claims 1 to 8 or the package of claim 10 for use in the treatment of otitis, preferably otitis media; upper respiratory tract infections, preferably tonsillitis or pharyngitis; lower respiratory tract infections, preferably bronchitis or pneumonia; diseases and inflammations of the oral cavity, preferably caries, oral mucositis, periodontitis, candidiasis, and/or oral lichen planus; halitosis; skin disorders, preferably acne and/or dermatitis; gastro-intestinal problems; allergies or immune diseases preferably allergic rhinitis, or mastitis in a subject.

12. The product of any one of claims 1 to 8 or the package of claim 10 suitable for supporting healthy mouth microflora, healthy upper and lower respiratory tract microflora, healthy skin, improving weight management, maintaining healthy gut microflora, healthy vaginal flora; maintaining a normal digestion or supporting healthy gut mobility, bowel movement and/or healthy stool frequency, stool consistency and/or form in a subject.

13. A method of producing a product which comprises at least probiotic microorganisms as active ingredient and isomaltulose as excipient, wherein the method comprises at least the following steps:
(i) mixing the probiotic microorganisms with isomaltulose and with one or more further excipients and optionally with one or more further active ingredients; and
(ii) homogenizing the mixture; and
(ii) compressing the mixture to a compressed product, wherein compression is performed with a pressure of 6 to 10 kN/compressed product, and optionally
(iii) analyzing the CFU/g amount and/or the water activity; and optionally the disintegration time, the average mass, the resistance to crushing, the appearance, the friability, and/or the water activity of the product.

14. The method of claim 13, wherein step (i) is composed of the following steps:
(a) mixing the isomaltulose with one or more further excipients, preferably with a lubricant/anti-caking agent, preferably with magnesium stearate;
(b) spraying a binder solution on the mixed excipients, preferably wherein the binder solution is composed of water and a binder, preferably wherein the binder is HMPC or pregelatinized starch, most preferably pregelatinized starch, thereby forming wet granules;
(c) drying the granules, preferably at a temperature of 40 ± 2°C for about 30 minutes;
(d) grinding the granules;
(e) adding the probiotic microorganisms to the grinded granules and optionally adding one or more further active ingredients.

15. The method of claim 13 or 14, wherein the product is the product of any one of claims 1 to 8.

## Patentansprüche

1. Produkt, das mindestens probiotische Mikroorganismen als Wirkstoff und Isomaltulose als Hilfsstoff umfasst, wobei das Produkt ein verpresstes Produkt ist, wobei das verpresste Produkt eine Lutschtablette ist, und wobei die probiotischen Mikroorganismen mit der Isomaltulose vermischt sind, wobei die Mischung homogenisiert ist, und wobei die Mischung zu dem verpressten Produkt verpresst ist, wobei das Verpressen mit einem Druck von 6 bis 10 kN pro verpresstem Produkt durchgeführt wird.

2. Produkt nach Anspruch 1, wobei die probiotischen Mikroorganismen Milchsäurebakterien sind.

3. Produkt nach Anspruch 1 oder 2, wobei die probiotischen Mikroorganismen der Gattung *Streptococcus, Lactobacillus, Lactococcus, Enterococcus* und/oder *Bifidobacterium* angehören, wobei die probiotischen Mikroorganismen vorzugsweise ausgewählt sind aus *Streptococcus salivarius,* vorzugsweise *Streptococcus salivarius* K12, *Streptococcus salivarius* ENT-K12 oder *Streptococcus salivarius* M18; *Lactobacillus rhamnosus,* vorzugsweise *Lactobacillus rhamnosus* LGG; *Lactobacillus casei*; *Lactobacillus paracasei; Lactobacillus fermentum*; *Lactobacillus crispatus*; *Lactobacillus plantarum; Bifidobacterium animalis subsp. lactis*; *Lactococcus lactis*; *Enterococcus faecalis*; *Lactobacillus reuteri*; jeweils einzeln oder in Kombination.

4. Produkt nach einem der Ansprüche 1 bis 3, das ferner einen oder mehrere Hilfsstoffe umfasst, vorzugsweise ein Schmiermittel und/oder ein Antiklumpmittel und/oder ein Bindemittel und/oder ein Aromastoff, wobei das Schmiermittel vorzugsweise Magnesiumstearat oder ein natürlicher Ersatz ist, wobei das Antiklumpmittel Siliciumdioxid, Tricalciumphosphat oder ein natürlicher Ersatz ist, wobei das Bindemittel HMPC oder ein natürlicher Ersatz ist und/oder wobei der Aromastoff ein Aroma ist, vorzugsweise Erdbeeraroma, und optional wobei das Produkt ferner einen natürlichen Süßstoff und/oder einen oder mehrere Wirkstoffe umfasst, vorzugsweise Vitamine, Mineralstoffe und/oder Fructooligosaccharide, wobei das Vitamin vorzugsweise Vitamin D3 ist.

5. Produkt nach einem der Ansprüche 1 bis 3, wobei das Produkt ferner umfasst:
(i) ein Schmiermittel/Antiklumpmittel, wobei das Schmiermittel/ Antiklumpmittel vorzugsweise Magnesiumstearat ist oder ein natürlicher Ersatz; einen Aromastoff, wobei der Aromastoff vorzugsweise ein Aroma ist, vorzugsweise Erdbeeraroma; und optional einen natürlichen Süßstoff und/oder einen oder mehrere Wirkstoffe, vorzugsweise ein Vitamin, vorzugsweise Vitamin D3; oder
(ii) ein Schmiermittel/Antiklumpmittel, wobei das Schmiermittel/ Antiklumpmittel vorzugsweise Magnesiumstearat oder ein natürlicher Ersatz; ein Bindemittel, wobei das Bindemittel vorzugsweise Hydroxypropylmethylcellulose (HMPC) ist oder ein natürlicher Ersatz; einen Aromastoff, wobei der Aromastoff vorzugsweise ein Aroma ist, vorzugsweise Erdbeeraroma; und optional einen natürlichen Süßstoff und/oder einen oder mehrere Wirkstoffe, vorzugsweise ein Vitamin, vorzugsweise Vitamin D3.

6. Produkt nach einem der Ansprüche 1 bis 3, wobei das Produkt ferner eine Reisextraktmischung als Schmiermittel/Antiklumpmittel, vorverkleisterte Maisstärke als Bindemittel, ein Aroma als Aromastoff, vorzugsweise Erdbeeraroma, einen natürlichen Süßstoff, vorzugsweise Stevia, und Vitamin D3 als weiteren Wirkstoff umfasst.

7. Produkt nach einem der Ansprüche 1 bis 6, wobei der Gehalt an Isomaltulose mindestens 80% beträgt, vorzugsweise zwischen 80% und 97%, noch bevorzugter zwischen 82% und 92%, und/oder wobei der Gehalt an probiotischen Mikroorganismen zwischen 0,6% und 20%, vorzugsweise zwischen 3% und 15% liegt; und/oder wobei das Produkt zwischen 5 mg und 160 mg, vorzugsweise zwischen 30 mg und 120 mg, insbesondere 40 mg, 50 mg, 60 mg, 100 mg oder 120 mg probiotische Mikroorganismen umfasst.

8. Produkt nach einem der Ansprüche 1 bis 7, das eine Zerfallszeit von mehr als 5 Minuten, vorzugsweise zwischen 10 und 30 Minuten, noch bevorzugter zwischen 10 und 11 Minuten aufweist und/oder eine Abriebfestigkeit von etwa 1,2 bis 1,5% aufweist, vorzugsweise etwa 1,25%, wobei das Produkt mindestens 80%, vorzugsweise zwischen 80% und 97%, noch bevorzugter zwischen 82% und 92% Isomaltulose umfasst und ferner umfassend
(i) ein Schmiermittel/Antiklumpmittel, einen Aromastoff und optional einen natürlichen Süßstoff und/oder einen oder mehrere Wirkstoffe; oder
(ii) ein Schmiermittel/Antiklumpmittel, ein Bindemittel, einen Aromastoff und optional einen natürlichen Süßstoff und/oder einen oder mehrere Wirkstoffe.

9. Verwendung von Isomaltulose als Hilfsstoff in einem Produkt nach einem der Ansprüche 1 bis 8.

10. Verpackung, umfassend das Produkt nach einem der Ansprüche 1 bis 8.

11. Produkt nach einem der Ansprüche 1 bis 8 oder die Verpackung nach Anspruch 10 zur Verwendung bei der Behandlung von Otitis, vorzugsweise Otitis media; Infektionen der oberen Atemwege, vorzugsweise Tonsillitis oder Pharyngitis; Infektionen der unteren Atemwege, vorzugsweise Bronchitis oder Pneumonie; Erkrankungen und Entzündungen der Mundhöhle, vorzugsweise Karies, orale Mukositis, Parodontitis, Candidiasis und/oder oraler Lichen planus; Halitosis; Hauterkrankungen, vorzugsweise Akne und/oder Dermatitis; gastrointestinale Probleme; Allergien oder Immunerkrankungen, vorzugsweise allergische Rhinitis, oder Mastitis bei einem Subjekt.

12. Produkt nach einem der Ansprüche 1 bis 8 oder die Verpackung nach Anspruch 10, geeignet zur Unterstützung einer gesunden Mundflora, einer gesunden Mikroflora der oberen und unteren Atemwege, gesunder Haut, zur Verbesserung des Gewichtsmanagements, zur Aufrechterhaltung einer gesunden Darmmikroflora, einer gesunden Vaginalflora; zur Aufrechterhaltung einer normalen Verdauung oder zur Unterstützung einer gesunden Darmbeweglichkeit, des Stuhlgangs und/oder einer gesunden Stuhlfrequenz, Stuhlkonsistenz und/oder -form bei einem Subjekt.

13. Verfahren zur Herstellung eines Produkts, das zumindest probiotische Mikroorganismen als Wirkstoff und Isomaltulose als Hilfsstoff umfasst, wobei das Verfahren zumindest folgende Schritte umfasst:
(i) Mischen der probiotischen Mikroorganismen mit Isomaltulose und mit einem oder mehreren weiteren Hilfsstoffen und optional mit einem oder mehreren weiteren Wirkstoffen; und
(ii) Homogenisieren der Mischung; und
(ii) Verpressen der Mischung zu einem verpressten Produkt, wobei das Verpressen mit einem Druck von 6 bis 10 kN pro verpresstem Produkt durchgeführt wird, und optional
(iii) Analyse der CFU/g-Menge und/oder der Wasseraktivität; und optional der Zerfallszeit, der mittleren Masse, der Bruchfestigkeit, des Aussehens, der Abriebfestigkeit und/oder der Wasseraktivität des Produkts.

14. Verfahren nach Anspruch 13, wobei Schritt (i) folgende Schritte umfasst:
(a) Mischen der Isomaltulose mit einem oder mehreren weiteren Hilfsstoffen, vorzugsweise einem Schmiermittel/Antiklumpmittel, vorzugsweise Magnesiumstearat;
(b) Versprühen einer Bindemittellösung auf die gemischten Hilfsstoffe, wobei die Bindemittellösung vorzugsweise aus Wasser und einem Bindemittel besteht, wobei das Bindemittel vorzugsweise HMPC oder vorverkleisterte Stärke ist, noch bevorzugter vorverkleisterte Stärke, wodurch feuchte Granulate gebildet werden;
(c) Trocknen der Granulate, vorzugsweise bei einer Temperatur von 40 ± 2°C für etwa 30 Minuten;
(d) Zerkleinern der Granulate;
(e) Zugabe der probiotischen Mikroorganismen zu den zerkleinerten Granulaten und optional Zugabe eines oder mehrerer weiterer Wirkstoffe.

15. Verfahren nach Anspruch 13 oder 14, wobei das Produkt ein Produkt nach einem der Ansprüche 1 bis 8 ist.

## Revendications

1. Un produit qui comprend au moins des micro-organismes probiotiques comme principe actif et de l'isomaltulose comme excipient, lequel produit est un produit comprimé, lequel produit comprimé est une pastille, et dans lequel les micro-organismes probiotiques sont mélangés avec l'isomaltulose, dans lequel le mélange est homogénéisé, et dans lequel le mélange est comprimé pour former le produit comprimé, la compression étant réalisée avec une pression de 6 à 10 kN/produit comprimé.

2. Le produit selon la revendication 1, dans lequel les micro-organismes probiotiques sont des bactéries lactiques.

3. Le produit selon la revendication 1 ou 2, dans lequel les micro-organismes probiotiques sont du genre *Streptococcus, Lactobacillus, Lactococcus, Enterococcus* et/ou *Bifidobacterium*, de préférence dans lequel les micro-organismes probiotiques sont sélectionnés dans le groupe constitué de *Streptococcus salivarius,* de préférence *Streptococcus salivarius* K12, *Streptococcus salivarius* ENT-K12 ou *Streptococcus salivarius* M18; *Lactobacillus rhamnosus,* de préférence *Lactobacillus rhamnosus* LGG; *Lactobacillus casei; Lactobacillus paracasei; Lactobacillus fermentum; Lactobacillus crispatus; Lactobacillus plantarum; Bifidobacterium animalis subsp. lactis, Lactococcus lactis; Enterococcus faecalis; Lactobacillus reuteri;* seuls ou en combinaison.

4. Le produit selon l'une quelconque des revendications 1 à 3, qui comprend en outre un ou plusieurs excipients, de préférence un lubrifiant et/ou un agent anti-agglomérant, et/ou un liant, et/ou un agent aromatisant, de préférence dans lequel le lubrifiant est du stéarate de magnésium ou un substitut naturel, dans lequel l'agent anti-agglomérant est du dioxyde de silicium, du phosphate tricalcique ou un substitut naturel, dans lequel le liant est de l'hydroxypropylméthylcellulose (HPMC) ou un substitut naturel, et/ou dans lequel l'agent aromatisant est un arôme, de préférence un arôme de fraise, et éventuellement dans lequel le produit comprend en outre un édulcorant naturel, et/ou un ou plusieurs principes actifs, de préférence des vitamines, des minéraux et/ou des fructooligosaccharides, de préférence dans lequel la vitamine est la vitamine D3.

5. Le produit selon l'une quelconque des revendications 1 à 3, dans lequel le produit comprend en outre
(i) un lubrifiant/un agent anti-agglomérant, de préférence dans lequel le lubrifiant/l'agent anti-agglomérant est du stéarate de magnésium ou un substitut naturel; un agent aromatisant, de préférence dans lequel l'agent aromatisant est un arôme, de préférence un arôme de fraise; et éventuellement un édulcorant naturel et/ou un ou plusieurs principes actifs, de préférence une vitamine, de préférence la vitamine D3; ou
(ii) un lubrifiant/un agent anti-agglomérant, de préférence dans lequel le lubrifiant/l'agent anti-agglomérant est du stéarate de magnésium ou un substitut naturel ; un liant, de préférence dans lequel le liant est de l'hydroxypropylméthylcellulose (HPMC) ou un substitut naturel ; un agent aromatisant, de préférence dans lequel l'agent aromatisant est un arôme, de préférence un arôme de fraise ; et éventuellement un édulcorant naturel, et/ou un ou plusieurs principes actifs, de préférence une vitamine, de préférence la vitamine D3.

6. Le produit selon l'une quelconque des revendications 1 à 3, dans lequel le produit comprend en outre un mélange d'extrait de riz comme lubrifiant/agent anti-agglomérant, de l'amidon de maïs prégélatinisé comme liant, un arôme comme agent aromatisant, de préférence un arôme de fraise, un édulcorant naturel, de préférence de la stévia, et de la vitamine D3 comme principe actif supplémentaire.

7. Le produit selon l'une quelconque des revendications 1 à 6, dans lequel la teneur en isomaltulose est d'au moins 80 %, de préférence entre 80 % et 97 %, plus préférentiellement entre 82 % et 92 %, et/ou dans lequel la teneur en micro-organismes probiotiques est comprise entre 0,6 % et 20 %, de préférence entre 3 % et 15 %; et/ou dans lequel le produit comprend entre 5 mg et 160 mg, de préférence entre 30 mg et 120 mg, plus préférentiellement 40 mg, 50 mg, 60 mg, 100 mg ou 120 mg de micro-organismes probiotiques.

8. Le produit selon l'une quelconque des revendications 1 à 7, qui présente un temps de désintégration de plus de 5 minutes, de préférence entre 10 et 30 minutes, plus préférentiellement entre 10 et 11 minutes et/ou qui présente une friabilité d'environ 1,2 à 1,5 %, de préférence d'environ 1,25 %, dans lequel le produit comprend au moins 80 %, de préférence entre 80 % et 97 %, plus préférentiellement entre 82 % et 92 % d'isomaltulose et comprend en outre
(i) un lubrifiant/un agent anti-agglomérant ; un agent aromatisant; et éventuellement un édulcorant naturel ; et/ou un ou plusieurs principes actifs; ou
(ii) un lubrifiant/un agent anti-agglomérant ; un liant ; un agent aromatisant; et éventuellement un édulcorant naturel ; et/ou un ou plusieurs principes actifs.

9. Utilisation de l'isomaltulose comme excipient dans un produit tel que défini dans l'une quelconque des revendications 1 à 8.

10. Un emballage comprenant le produit selon l'une quelconque des revendications 1 à 8.

11. Le produit selon l'une quelconque des revendications 1 à 8 ou l'emballage selon la revendication 10 pour une utilisation dans le traitement de l'otite, de préférence de l'otite moyenne; des infections des voies respiratoires supérieures, de préférence de l'amygdalite ou de la pharyngite; des infections des voies respiratoires inférieures, de préférence de la bronchite ou de la pneumonie; des maladies et inflammations de la cavité buccale, de préférence de la carie, de la mucite buccale, de la parodontite, de la candidose et/ou du lichen plan buccal; de l'halitose; des troubles cutanés, de préférence de l'acné et/ou de la dermatite; des problèmes gastro-intestinaux; des allergies ou des maladies immunitaires, de préférence de la rhinite allergique, ou de la mastite chez un sujet.

12. Le produit selon l'une quelconque des revendications 1 à 8 ou l'emballage selon la revendication 10 adapté pour soutenir la microflore buccale saine, la microflore saine des voies respiratoires supérieures et inférieures, la peau saine, améliorer la gestion du poids, maintenir une microflore intestinale saine, une flore vaginale saine; maintenir une digestion normale ou soutenir la motilité intestinale saine, le transit intestinal et/ou la fréquence normale des selles, la consistance et/ou la forme des selles chez un sujet.

13. Un procédé de production d'un produit qui comprend au moins des micro-organismes probiotiques comme principe actif et de l'isomaltulose comme excipient, dans lequel le procédé comprend au moins les étapes suivantes:
(i) mélange des micro-organismes probiotiques avec de l'isomaltulose et avec un ou plusieurs excipients supplémentaires et éventuellement avec un ou plusieurs principes actifs supplémentaires; et
(ii) homogénéisation du mélange; et
(ii) compression du mélange pour former un produit comprimé, la compression étant réalisée avec une pression de 6 à 10 kN/produit comprimé, et éventuellement
(iii) analyse de la quantité en UFC/g et/ou de l'activité de l'eau ; et éventuellement du temps de désintégration, de la masse moyenne, de la résistance à l'écrasement, de l'aspect, de la friabilité, et/ou de l'activité de l'eau du produit.

14. Le procédé selon la revendication 13, dans lequel l'étape (i) est composée des étapes suivantes:
(a) mélange de l'isomaltulose avec un ou plusieurs excipients supplémentaires, de préférence avec un lubrifiant/agent anti-agglomérant, de préférence avec du stéarate de magnésium;
(b) pulvérisation d'une solution de liant sur les excipients mélangés, de préférence dans laquelle la solution de liant est composée d'eau et d'un liant, de préférence dans laquelle le liant est de l'hydroxypropylméthylcellulose (HPMC) ou de l'amidon prégélatinisé, plus préférentiellement de l'amidon prégélatinisé, formant ainsi des granulés humides;
(c) séchage des granulés, de préférence à une température de 40 ±2^∘C pendant environ 30 minutes;
(d) broyage des granulés;
(e) ajout des micro-organismes probiotiques aux granulés broyés et éventuellement ajout d'un ou plusieurs principes actifs supplémentaires.

15. Le procédé selon la revendication 13 ou 14, dans lequel le produit est le produit selon l'une quelconque des revendications 1 à 8.
